(19) Europäisches Patentamt
European Patent Office
Office européen des brevets

(11) EP 4 165 040 B1

(12) # EUROPEAN PATENT SPECIFICATION

(45) Date of publication and mention of the grant of the patent:
**27.08.2025 Bulletin 2025/35**

(21) Application number: **21737276.2**

(22) Date of filing: **09.06.2021**

(51) International Patent Classification (IPC):
***C07D 471/04** (2006.01)* ***C07D 487/04** (2006.01)*
***A61P 35/00** (2006.01)* ***A61K 31/519** (2006.01)*

(52) Cooperative Patent Classification (CPC):
**C07D 471/04; A61P 35/00; C07D 487/04**

(86) International application number:
**PCT/US2021/036679**

(87) International publication number:
**WO 2021/252679 (16.12.2021 Gazette 2021/50)**

(54) **2-AMINOQUINAZOLINONE DERIVATIVES AS METHIONINE ADENOSYLTRANSFERASE 2A INHIBITORS**

2-AMINOCHINAZOLINONDERIVATE ALS METHIONIN-ADENOSYLTRANSFERASE 2A-HEMMER

DÉRIVÉS DE 2-AMINOQUINAZOLINONE COMME INHIBITEURS DE LA MÉTHIONINE ADÉNOSYLTRANSFÉRASE 2A

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2020 US 202063037109 P**

(43) Date of publication of application:
**19.04.2023 Bulletin 2023/16**

(73) Proprietor: **Ideaya Biosciences, Inc.**
**South San Francisco, CA 94080 (US)**

(72) Inventors:
- **STEEL, Richard**
  **South San Francisco, California 94080 (US)**
- **FLEURY, Melissa**
  **South San Francisco, California 94080 (US)**
- **BARSANTI, Paul A.**
  **South San Francisco, California 94080 (US)**

(74) Representative: **Eisenführ Speiser**
**Patentanwälte Rechtsanwälte PartGmbB**
**Postfach 10 60 78**
**28060 Bremen (DE)**

(56) References cited:
**WO-A1-2018/039972** **WO-A1-2019/191470**
**WO-A1-2019/213516**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### CROSS-REFERENCES TO RELATED APPLICATIONS

This application claims the benefit under 35 U.S.C. 119(e) of U.S. Provisional Application No. 63/037,109, filed on June 10, 2020.

### STATEMENT AS TO RIGHTS TO INVENTIONS MADE UNDER FEDERALLY SPONSORED RESEARCH AND DEVELOPMENT

NOT APPLICABLE

### REFERENCE TO A "SEQUENCE LISTING," A TABLE, OR A COMPUTER PROGRAM LISTING APPENDIX SUBMITTED ON A COMPACT DISK

NOT APPLICABLE

### BACKGROUND OF THE INVENTION

Cancer is a leading cause of death throughout the world. A limitation of prevailing therapeutic approaches, e.g. chemotherapy and immunotherapy is that their cytotoxic effects are not restricted to cancer cells and adverse side effects can occur within normal tissues. Consequently, novel strategies are needed to better target cancer cells.

Synthetic lethality arises when a combination of deficiencies in the expression of two or more genes leads to cell death, whereas a deficiency in only one of these genes does not. The concept of synthetic lethality originates from studies in drosophila model systems in which a combination of mutations in two or more separate genes leads to cell death (in contrast to viability, which occurs when only one of the genes is mutated or deleted). More recently, a multitude of studies have explored maladaptive genetic changes in cancer cells that render them vulnerable to synthetic-lethality approaches. These tumor-specific genetic defects lead to the use of targeted agents that induce the death of tumor cells while sparing normal cells.

Methionine adenosyltransferase 2A (MAT2A) is an enzyme that utilizes methionine (Met) and adenosine triphosphate (ATP) to generate s-adenosyl methionine (SAM). SAM is a primary methyl donor in cells used to methylate several substrates including DNA, RNA and proteins. One methylase that utilizes SAM as a methyl donor, is protein arginine N-methyltransferase 5 (PRMT5). While SAM is required for PRMT5 activity, PRMT5 is competitively inhibited by 5'methylthioadenosine (MTA). Since MTA is part of the methionine salvage pathway, cellular MTA levels stay low in a process initiated by methylthioadenosine phosphorylase (MTAP).

MTAP is in a locus on chromosome 9 that is often deleted in cells of patients with cancers from several tissues of origin including central nervous system, pancreas, esophageal, bladder and lung (cBioPortal database). Loss of MTAP results in the accumulation of MTA making MTAP-deleted cells more dependent on SAM production, and thus MAT2A activity, compared to cells that express MTAP. In an shRNA cell-line screen across approximately 400 cancer cell lines, MAT2A knockdown resulted in the loss of viability in a larger percentage of MTAP-deleted cells compare to MTAP WT cells (*see* McDonald et. al. 2017 Cell 170, 577-592). Furthermore, inducible knockdown of MAT2A protein decreased tumor growth *in vivo* (*see* Marjon et. al., 2016 Cell Reports 15(3), 574-587). These results indicate that MAT2A inhibitors may provide a novel therapy for cancer patients including those with MTAP-deleted tumors. MAT2A inhibitors are disclosed, for instance, in WO 2018/039972 and WO 2019/191470.

### SUMMARY

Disclosed herein are certain 2-aminoquinazoline derivatives that are methionine adenosyltransferase 2A (MAT2A) inhibitors. Also disclosed are pharmaceutical compositions comprising such compounds and such compounds for use in methods of treating cancer treatable by inhibition of MAT2A, including cancers characterized by reduced or absence of methylthioadenosine phosphorylase (MTAP) activity. Methods of treatment disclosed herein are not part of the invention. Passages referring to methods of treatment are to be understood as relating to the compounds of the invention for use in these methods of treatment.

In a first aspect, provided herein is a compound of Formula (A)

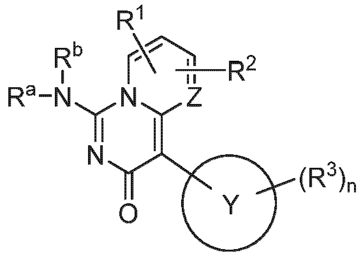

(A)

or a pharamaceutically acceptable salt thererof, wherein Z, $R^1$, $R^2$, $R^a$, $R^b$, $R^3$, n and ring Y are as defined herein.

In a second aspect, provided is a compound of Formula (I):

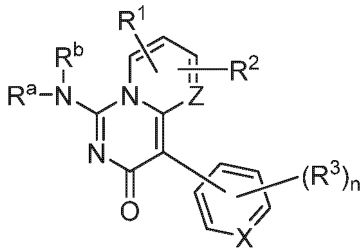

(I)

or a pharmaceutically acceptable salt thereof, wherein

Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, and $C_{1-6}$ haloalkyl; or

$R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl;
each $X^1$ is $C_{1-6}$ alkylene;

X is CH, $CR^3$, or N;
each $R^3$ is independently selected from the group consisting of halo, $C_{1-4}$ alkyl, and $C_{1-4}$ haloalkyl; and
the subscript n is 0, 1 or 2.

In a third aspect, provided is a pharmaceutical composition comprising a compound of Formula (A), Formula (I), a subembodiment described herein, or a pharmaceutically acceptable salt thereof at least one pharmaceutically acceptable excipient.

In a fourth aspect, provided is a method for treating cancer mediated by MAT2A in a patient comprising administering to the patient a therapeutically effective amount of a compound of Formula (A), Formula (I), a subembodiment described herein, or a pharmaceutically acceptable salt thereof. In first embodiment of the third aspect, the patient is in recognized need of such treatment. In second embodiment of the third aspect and first embodiment contained therein, the compound of Formula (A), Formula (I), a subembodiment described herein, or a pharmaceutically acceptable salt thereof is administered in a pharmaceutical composition. In a third embodiment of the third aspect and first and second embodiments contained therein, the cancer is mediated by overexpression of MAT2A.

In a fifth aspect, provided is a method of treating a MTAP null cancer in a patient comprising administering to the patient a therapeutically effective amount of a compound of Formula (A), Formula (I), a subembodiment described herein, or a pharmaceutically acceptable salt thereof. In first embodiment of the fourth aspect, the patient is in recognized need of such treatment. In second embodiment of the fourth aspect and first embodiment contained therein, the compound of Formula (A), Formula (I), a subembodiment described herein, or a pharmaceutically acceptable salt thereof is administered in a pharmaceutical composition.

In a sixth aspect of the description, provided is a method for inhibiting the synthesis of S-adenosyl methionine

(SAM) from methionine and ATP by MAT2A in a cell comprising contacting the cell with an effective amount of a compound of Formula (A), Formula (I), a subembodiment described herein, or a pharmaceutically acceptable salt thereof.

In a seventh aspect, provided is a method for treating a cancer in a patient, wherein the cancer is characterized by a reduction or absence of methylthioadenosine phosphorylase (MTAP) gene expression, the absence of the MTAP gene, or reduced function of MTAP protein, comprising administering to the subject a therapeutically effective amount of a compound of Formula (A), Formula (I), a subembodiment described herein, or a pharmaceutically acceptable salt thereof optionally in a pharmaceutical composition.

In an eighth aspect, provided is a compound of Formula (A), Formula (I) a subembodiment described herein, or a pharmaceutically acceptable salt thereof for inhibiting the synthesis of S-adenosyl methionine (SAM) from methionine and ATP by MAT2A in a cell.

In an ninth aspect, provided is a compound of Formula (A), Formula (I), a subembodiment described herein, or a pharmaceutically acceptable salt thereof for use in the treatment of cancer in a patient, wherein the disease is mediated by the overexpression of MAT2A.

In a tenth aspect, provided is a compound of Formula (A), Formula (I), a subembodiment described herein, or a pharmaceutically acceptable salt thereof for use in the treatment a cancer in a patient, wherein the cancer is characterized by a reduction or absence of methylthioadenosine phosphorylase (MTAP) gene expression, the absence of the MTAP gene, or reduced function of MTAP protein.

In an eleventh aspect, provided is a method for treating a cancer in a patient, wherein the cancer is characterized by a reduction or absence of methylthioadenosine phosphorylase (MTAP) gene expression, the absence of the MTAP gene, reduced level of MTAP protein, or reduced function of MTAP protein, comprising administering to the subject a therapeutically effective amount of a compound of Formula (A), Formula (I), a subembodiment described herein, or a pharmaceutically acceptable salt thereof optionally in a pharmaceutical composition.

In a twelfth aspect, provided is a compound of Formula (A), Formula (I), a subembodiment described herein, or a pharmaceutically acceptable salt thereof for use in the treatment a cancer in a patient, wherein the cancer is characterized by a reduction or absence of methylthioadenosine phosphorylase (MTAP) gene expression, the absence of the MTAP gene, reduced level of MTAP protein, or reduced function of MTAP protein.

## DETAILED DESCRIPTION

Before the present invention is further described, it is to be understood that the invention is not limited to the particular embodiments set forth herein, and it is also to be understood that the terminology used herein is for the purpose of describing particular embodiments only, and is not intended to be limiting.

The singular forms "a," "an," and "the" as used herein and in the appended claims include plural referents unless the context clearly dictates otherwise. It is further noted that the claims may be drafted to exclude any optional element. As such, this statement is intended to serve as antecedent basis for use of such exclusive terminology such as "solely," "only" and the like in connection with the recitation of claim elements, or use of a "negative" limitation.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention. Unless defined otherwise, all technical and scientific terms used herein have the same meaning as commonly understood by one of ordinary skill in the art to which this invention belongs.

The publications discussed herein are provided solely for their disclosure prior to the filing date of the present application. Further, the dates of publication provided may be different from the actual publication dates, which may need to be independently confirmed.

### Definitions:

Unless otherwise stated, the following terms used in the specification and claims are defined for the purposes of this Application and have the following meaning:

"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, butyl, pentyl, and the like. It will be recognized by a person skilled in the art that the term "alkyl" may include "alkylene" groups.

"Alkylene" means a linear saturated divalent hydrocarbon radical of one to six carbon atoms or a branched saturated divalent hydrocarbon radical of three to six carbon atoms unless otherwise stated e.g., methylene, ethylene, propylene, 1-methylpropylene, 2-methylpropylene, butylene, pentylene, and the like.

"Alkoxy" means a -OR radical where R is alkyl as defined above, e.g., methoxy, ethoxy, propoxy, or 2-propoxy, *n-, iso-,* or *tert*-butoxy, and the like.

"Haloalkoxy" means an alkoxy radical, as defined above, which is substituted with one to five halogen atoms, such as fluorine or chlorine, including those substituted with different halogens, e.g., $-OCH_2Cl$, $-OCF_3$, $-OCHF_2$, $-OCH_2CF_3$, $-OCF_2CF_3$, $-OCF(CH_3)_2$, and the like.

"Aryl" means a monovalent monocyclic or bicyclic aromatic hydrocarbon radical of 6 to 10 ring atoms e.g., phenyl or naphthyl.

"Heteroaryl" means a monovalent monocyclic or bicyclic aromatic radical of 5 to 10 ring atoms, unless otherwise stated, where one or more, (in one embodiment, one, two, or three), ring atoms are heteroatom selected from N, O, or S, the remaining ring atoms being carbon. Non-limiting examples of heteroaryl groups include pyridyl, pyridazinyl, pyrazinyl, pyrimindinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl, thienyl, and the like. As defined herein, the terms "heteroaryl" and "aryl" are mutually exclusive. When the heteroaryl ring contains 5- or 6 ring atoms it is also referred to herein as 5-or 6-membered heteroaryl.

"Cycloalkyl" means a monocyclic monovalent hydrocarbon radical of three to eight carbon atoms which may be saturated or contains one double bond. Cycloalkyl may be unsubstituted or substituted with one or two substituents independently selected from alkyl, halo, alkoxy, hydroxy, or cyano. Examples include, but are not limited to, cyclopropyl, cyclobutyl, cyclopentyl, cyclohexyl, 1-cyanocycloprop-1-yl, 1-cyanomethylcycloprop-1-yl, 3-fluorocyclohexyl, and the like. When cycloalkyl contains a double bond, it may be referred to herein as cycloalkenyl.

"Halo" means fluoro, chloro, bromo, or iodo, preferably fluoro or chloro.

"Haloalkyl" means alkyl radical as defined above, which is substituted with one to five halogen atoms, such as fluorine or chlorine, including those substituted with different halogens, e.g., $-CH_2Cl$, $-CF_3$, $-CHF_2$, $-CH_2CF_3$, $-CF_2CF_3$, $-CF(CH_3)_2$, and the like. When the alkyl is substituted with only fluoro, it can be referred to in this Application as fluoroalkyl.

"Heterocycloalkyl" means a saturated or unsaturated monovalent monocyclic group of 4 to 8 ring atoms in which one or two ring atoms are heteroatom selected from N, O, or $S(O)_n$, where n is an integer from 0 to 2, the remaining ring atoms being C. Additionally, one or two ring carbon atoms in the heterocycloalkyl ring can optionally be replaced by a -CO- group. More specifically the term heterocycloalkyl includes, but is not limited to, azetidinyl, oxetanyl, pyrrolidino, piperidino, homopiperidino, 2-oxopyrrolidinyl, 2-oxopiperidinyl, morpholino, piperazino, tetrahydro-pyranyl, thiomorpholino, and the like. When the heterocycloalkyl ring is unsaturated it can contain one or two ring double bonds provided that the ring is not aromatic. When heterocycloalkyl contains at least one nitrogen atom, it may be referred to herein as heterocycloamino.

"Oxo," as used herein, alone or in combination, refers to =(O).

"Pharmaceutically acceptable salts" as used herein is meant to include salts of the active compounds which are prepared with relatively nontoxic acids or bases, depending on the particular substituents found on the compounds described herein. When compounds disclosed herein contain relatively acidic functionalities, base addition salts can be obtained by contacting the neutral form of such compounds with a sufficient amount of the desired base, either neat or in a suitable inert solvent. Examples of salts derived from pharmaceutically-acceptable inorganic bases include aluminum, ammonium, calcium, copper, ferric, ferrous, lithium, magnesium, manganic, manganous, potassium, sodium, zinc and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogen carbonic, phosphoric, monohydrogen phosphoric, dihydrogen phosphoric, sulfuric, monohydrogen sulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al, "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in

certain physical properties, such as solubility in polar solvents, but otherwise the salts are equivalent to the parent form of the compound for the purposes of the present invention.

The present disclosure also includes protected derivatives of compounds of the present disclosure. Protected derivatives are not part of the invention. For example, when compounds of the present disclosure contain groups such as hydroxy, carboxy, thiol or any group containing a nitrogen atom(s), these groups can be protected with a suitable protecting groups. A comprehensive list of suitable protective groups can be found in T.W. Greene, Protective Groups in Organic Synthesis, 5th Ed., John Wiley & Sons, Inc. (2014). The protected derivatives of compounds of the present disclosure can be prepared by methods well known in the art.

The present disclosure also includes prodrugs of the compound of Formula (A), Formula (I), a subembodiment described herein, or a pharmaceutically acceptable salt thereof. Prodrugs are not part of the invention. Prodrugs of the compounds described herein are those compounds that readily undergo chemical changes under physiological conditions to provide the compounds of the present invention. An example, without limitation, of a prodrug would be a compound which is administered as an ester (the "prodrug"), but then is metabolically hydrolyzed to the carboxylic acid, the active entity. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Certain compounds of Formula (A), Formula (I) or a subembodiment described herein may exist in multiple crystalline or amorphous forms. In general, all physical forms are equivalent for the uses contemplated by the present disclosure and are intended to be within the scope of the present disclosure.

Certain compounds of Formula (A), Formula (I) or a subembodiment described herein possess asymmetric carbon atoms (optical centers) or double bonds; the racemates, diastereomers, geometric isomers, and separate enantiomers are all intended to be encompassed within the scope of the present invention. Also within the scope of the present disclosure are atropisomers (isomers based on axial chirality resulting from restricted rotation in the molecule) of Formula (A), Formula (I) or a subembodiment described herein. When a stereochemical depiction is shown, it is meant to refer the compound in which one of the isomers is present and substantially free of the other isomer. 'Substantially free of' another isomer indicates at least an 80/20 ratio of the two isomers, more preferably 90/10, or 95/5 or more. In some embodiments, one of the isomers will be present in an amount of at least 99%.

The compounds of Formula (A), Formula (I) or a subembodiment described herein may also contain unnatural amounts of isotopes at one or more of the atoms that constitute such compounds. Unnatural amounts of an isotope may be defined as ranging from the amount found in nature to an amount 100% of the atom in question. that differ only in the presence of one or more isotopically enriched atoms. Exemplary isotopes that can be incorporated into compounds of the present invention, such as a compound of Formula (A), Formula (I), a subembodiment described herein (including specific compounds) include isotopes of hydrogen, carbon, nitrogen, oxygen, phosphorus, sulfur, fluorine, chlorine, and iodine, such as $^{2}H$, $^{3}H$, $^{11}C$, $^{13}C$, $^{14}C$, $^{13}N$, $^{15}N$, $^{15}O$, $^{17}O$, $^{18}O$, $^{32}P$, $^{33}P$, $^{35}S$, $^{18}F$, $^{36}Cl$, $^{123}I$, and $^{125}1$, respectively. Isotopically-labeled compounds (e.g., those labeled with $^{3}H$ and $^{14}C$) can be useful in compound or substrate tissue distribution assays. Tritiated (i.e., $^{3}H$) and carbon-14 (i.e., $^{14}C$) isotopes can be useful for their ease of preparation and detectability. Further, substitution with heavier isotopes such as deuterium (i.e., $^{2}H$) may afford certain therapeutic advantages resulting from greater metabolic stability (e.g., increased in vivo half-life or reduced dosage requirements). In some embodiments, in compounds disclosed herein, including in Table 1 below one or more hydrogen atoms are replaced by $^{2}H$ or $^{3}H$, or one or more carbon atoms are replaced by $^{13}C$- or $^{14}C$-enriched carbon. Positron emitting isotopes such as $^{15}O$, $^{13}N$, $^{11}C$, and $^{15}F$ are useful for positron emission tomography (PET) studies to examine substrate receptor occupancy. Isotopically labeled compounds can generally be prepared by following procedures analogous to those disclosed in the Schemes or in the Examples herein, by substituting an isotopically labeled reagent for a non-isotopically labeled reagent.

"Pharmaceutically acceptable carrier or excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

"About," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass $\pm$ 10%, preferably $\pm$ 5%, the recited value and the range is included.

"Disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

"Patient" is generally synonymous with the term "subject" and as used herein includes all mammals including humans. Examples of patients include humans, livestock such as cows, goats, sheep, pigs, and rabbits, and companion

animals such as dogs, cats, rabbits, and horses. Preferably, the patient is a human.

"In need of treatment" as used herein refers to a judgment made by a physician or other caregiver that a subject requires or will benefit from treatment. This judgment is made based on a variety of factors that are in the realm of the physician's or caregiver's expertise.

"Administration", "administer" and the like, as they apply to, for example, a patient, cell, tissue, organ, or biological fluid, refer to contact of, for example, a compound of Formula (A), Formula (I), a pharmaceutical composition comprising same, or a diagnostic agent to the subject, cell, tissue, organ, or biological fluid. In the context of a cell, administration includes contact (e.g., in vitro or ex vivo) of a reagent to the cell, as well as contact of a reagent to a fluid, where the fluid is in contact with the cell.

"Therapeutically effective amount" as used herein means the amount of a compound of Formula (A), Formula (I) or a subembodiment described herein and/or a pharmaceutically acceptable salt thereof that, when administered to a patient for treating a disease either alone or as part of a pharmaceutical composition and either in a single dose or as part of a series of doses, is sufficient to affect such treatment for the disease. The "therapeutically effective amount" will vary depending on the compound, the disease and its severity and the age, weight, etc., of the mammal to be treated. The therapeutically effective amount can be ascertained by measuring relevant physiological effects, and it can be adjusted in connection with the dosing regimen and diagnostic analysis of the subject's condition, and the like. By way of example, measurement of the serum level of a compound of Formula (A), Formula (I) or a subembodiment described herein (or, e.g., a metabolite thereof) at a particular time post-administration may be indicative of whether a therapeutically effective amount has been used.

"Treating" or "treatment" of a disease includes:

(1) preventing the disease, i.e. causing the clinical symptoms of the disease not to develop in a mammal that may be exposed to or predisposed to the disease but does not yet experience or display symptoms of the disease;
(2) inhibiting the disease, i.e., arresting or reducing the development of the disease or its clinical symptoms; or
(3) relieving the disease, i.e., causing regression of the disease or its clinical symptoms.

"Inhibiting", "reducing," or any variation of these terms in relation of MAT2A, includes any measurable decrease or complete inhibition to achieve a desired result. For example, there may be a decrease of about, at most about, or at least about 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, reduction of MAT2A activity compared to its normal activity.

**Compounds of Formula (I)**

In some aspects, provided herein is a compound having the Formula (A)

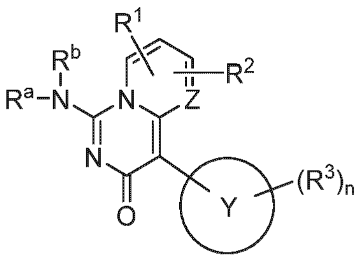

(A)

or a pharmaceutically acceptable salt thereof, wherein

Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, $C_{1-6}$ haloalkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, $C_{1-6}$ haloalkyl and -C(O)-$C_{1-4}$ alkyl; or

$R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, -$OR^x$, and -$X^1$-$OR^x$, and wherein each $R^x$ is

selected from the group consisting of H, $C_{1-4}$ alkyl, and $C_{1-4}$ haloalkyl;
each $X^1$ is $C_{1-6}$ alkylene;

ring Y is a phenyl or a 5 to 6 membered heteroaryl comprising 1 to 3 heteroatom ring vertices independently selected from the group consisting of N, O, and S;
each $R^3$ is independently selected from the group consisting of halo, cyano, $C_{1-4}$ alkoxy, $C_{1-4}$ haloalkoxy, -OH, $C_{1-4}$ alkyl, and $C_{1-4}$ haloalkyl; and
the subscript n is 0, 1 or 2.

In some embodiments, ring Y in Formula (A) is phenyl.

In some embodiments ring Y in Formula (A) is a 5 membered heteroaryl comprising 1 to 3 heteroatom ring vertices independently selected from the group consisting of N, O, and S. In some embodiments, ring Y in Formula (A) is a 6 membered heteroaryl comprising 1 to 3 heteroatom ring vertices independently selected from the group consisting of N, O, and S.

In some embodiments, ring Y in Formula (A) is selected from the group consisting of pyrroylyl, pyrazolyl, and triazolyl. In some embodiments ring Y in Formula (A) is pyrazolyl.

In some embodiments, ring Y in Formula (A) is selected from the group consisting of pyridyl, pyridazinyl, pyrazinyl, pyrimindinyl, and triazinyl. In some embodiments, ring Y in Formula (A) is pyridyl. In some embodiments, ring Y in Formula (A) is pyrazinyl.

In some aspects, provided herein are compounds having the Formula (I)

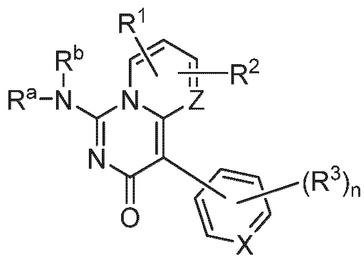

(I)

or a pharmaceutically acceptable salt thereof, wherein

Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, and $C_{1-6}$ haloalkyl; or

$R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl;
each $X^1$ is $C_{1-6}$ alkylene;

X is CH, $CR^3$, or N;
each $R^3$ is independently selected from the group consisting of halo, cyano, $C_{1-4}$ alkoxy, $C_{1-4}$ haloalkoxy, -OH, $C_{1-4}$ alkyl, and $C_{1-4}$ haloalkyl; and
the subscript n is 0, 1 or 2.

In some aspects, provided herein are compounds having the Formula (I)

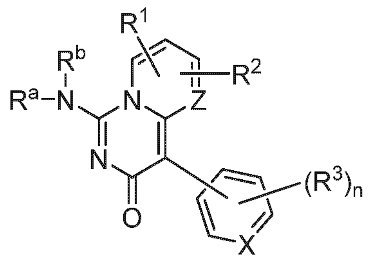

(I)

or a pharmaceutically acceptable salt thereof, wherein

Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, and $C_{1-6}$ haloalkyl; or

$R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl;
each $X^1$ is $C_{1-6}$ alkylene;

X is CH, $CR^3$, or N;
each $R^3$ is independently selected from the group consisting of halo, $C_{1-4}$ alkyl, and $C_{1-4}$ haloalkyl; and
the subscript n is 0, 1 or 2.

In some embodiments, the compounds described herein are represented by Formula (Ia)

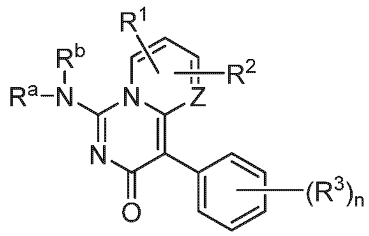

(Ia)

or a pharmaceutically acceptable salt thereof.

In some embodiments, the compounds described herein are represented by Formula (Ib)

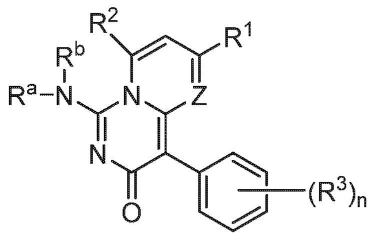

(Ib)

or a pharmaceutically acceptable salt thereof.

In some embodiments, the compounds described herein are represented by Formula (Ibb)

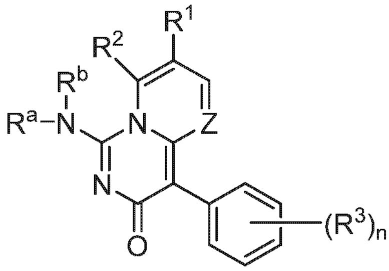

(Ibb)

or a pharmaceutically acceptable salt thereof.

In some embodiments, the compounds described herein are represented by Formula (Ic)

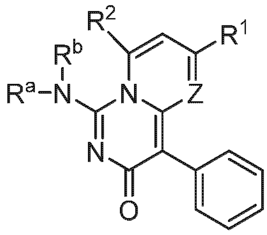

(Ic)

or a pharmaceutically acceptable salt thereof.

In some embodiments, the compounds described herein are represented by Formula (Id)

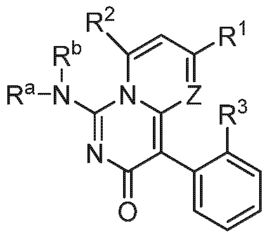

(Id)

or a pharmaceutically acceptable salt thereof.

In some embodiments, the compounds described herein are represented by Formula (Ie)

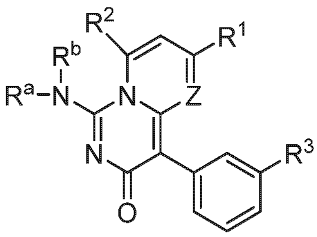

(Ie)

or a pharmaceutically acceptable salt thereof.

In some embodiments, the compounds described herein are represented by Formula (If)

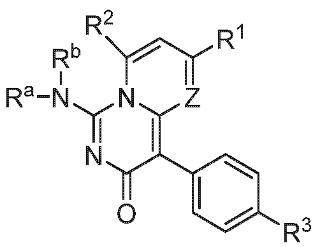

(If)

or a pharmaceutically acceptable salt thereof.

In some embodiments of Formula (A), Formula (I) and relevant subembodiments thereof, Z is CH. In some embodiments of Formula (A), Formula (I) and relevant subembodiments thereof, Z is N.

In some embodiments of Formula (A), Formula (I) and relevant subembodiments thereof, $R^1$ is selected from the group consisting of $C_{1-6}$ alkyl, $C_{1-6}$ haloalkyl, halo, $C_{1-6}$ alkoxy, $C_{1-6}$ haloalkoxy, and $C_{3-8}$ cycloalkyl, wherein the cycloalkyl group is substituted with from 0 to 2 groups selected from the group consisting of $C_{1-4}$ alkyl and halo. In some embodiments of Formula (A), Formula (I) and relevant subembodiments thereof, $R^1$ is selected from the group consisting of $C_{1-2}$ alkyl, $C_{1-2}$ haloalkyl, and halo. In some embodiments of Formula (A), Formula (I) and relevant subembodiments thereof, $R^1$ is selected from the group consisting of methyl, trifluoromethyl, chloro, bromo, fluoro, methoxy, trifluoromethoxy, and cyclopropyl. In some embodiments of Formula (A), Formula (I) and relevant subembodiments thereof, $R^1$ is selected from the group consisting of methyl, trifluoromethyl, chloro, bromo, fluoro, and cyclopropyl. In some embodiments of Formula (A), Formula (I) and relevant subembodiments thereof, $R^1$ is H.

In some embodiments of Formula (A), Formula (I) and relevant subembodiments thereof, $R^1$ is selected from the group consisting of $C_{1-6}$ alkyl, $C_{1-6}$ haloalkyl, halo, and $C_{3-8}$ cycloalkyl, wherein the cycloalkyl group is substituted with from 0 to 2 groups selected from the group consisting of $C_{1-4}$ alkyl and halo. In some embodiments of Formula (A), Formula (I) and relevant subembodiments thereof, $R^1$ is selected from the group consisting of $C_{1-2}$ alkyl, $C_{1-2}$ haloalkyl, and halo. In some embodiments of Formula (A), Formula (I) and relevant subembodiments thereof, $R^1$ is selected from the group consisting of methyl, trifluoromethyl, chloro, bromo, fluoro, and cyclopropyl. In some embodiments of Formula (A), Formula (I) and relevant subembodiments thereof, $R^1$ is H.

In some embodiments of Formula (A), Formula (I) and relevant subembodiments thereof, $R^1$ is methyl. In some embodiments of Formula (A), Formula (I) and relevant subembodiments thereof, $R^1$ is trifluoromethyl. In some embodiments of Formula (A), Formula (I) and relevant subembodiments thereof, $R^1$ is chloro. In some embodiments of Formula (A), Formula (I) and relevant subembodiments thereof, $R^1$ is fluoro. In some embodiments of Formula (A), Formula (I) and relevant subembodiments thereof, $R^1$ is bromo. In some embodiments of Formula (A), Formula (I) and relevant subembodiments thereof, $R^1$ is cyclopropyl.

In some embodiments of Formula (A), Formula (I) and relevant subembodiments thereof, $R^2$ is selected from the group consisting of H, $C_{1-2}$ alkyl, halo, and $C_{1-2}$ alkoxy. In some embodiments of Formula (A), Formula (I) and relevant subembodiments thereof, $R^2$ is selected from the group consisting of H and methoxy.

In some embodiments of Formula (A), Formula (I) and relevant subembodiments thereof, $R^2$ is H. In some embodiments of Formula (A), Formula (I) and relevant subembodiments thereof, $R^2$ is methoxy. In some embodiments of Formula (A), Formula (I) and relevant subembodiments thereof, $R^2$ is cyano.

In some embodiments of Formula (A), Formula (I) and relevant subembodiments thereof, $R^a$ and $R^b$ are each independently selected from the group consisting of H, $C_{1-6}$ alkyl, and $C_{1-6}$ haloalkyl. In some embodiments of Formula (A), Formula (I) and relevant subembodiments thereof, $R^a$ and $R^b$ are each independently selected from the group consisting of H, $C_{1-2}$ alkyl, and $C_{1-2}$ haloalkyl. In some embodiments of Formula (A), Formula (I) and relevant subembodiments thereof, $R^a$ and $R^b$ are each H. In some embodiments of Formula (A), Formula (I) and relevant subembodiments thereof, $R^a$ and $R^b$ are each methyl. In some embodiments of Formula (A), Formula (I) and relevant subembodiments thereof, $R^a$ is H; and $R^b$ is methyl.

In some embodiments of Formula (A), Formula (I) and relevant subembodiments thereof, $R^a$ and $R^b$ together with the nitrogen to which they are attached combine to form a 4-to 6-membered heterocycloalkyl ring comprising 0 to 2 additional heteroatoms independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl; and each $X^1$ is $C_{1-6}$ alkylene.

In some embodiments of Formula (A), Formula (I) and relevant subembodiments thereof, $R^a$ and $R^b$ together with the nitrogen to which they are attached combine to form a 4-to 6-membered heterocycloalkyl ring, wherein the 4- to 6-membered heterocycloalkyl is substituted with 0 to 2 moieties independently selected from the group consisting of $C_{1-4}$ alkyl, $-OR^x$, and $-X^1-OR^x$, wherein each $R^x$ is selected from the group consisting of H, $C_{1-4}$ alkyl, and $C_{1-4}$ haloalkyl; and each $X^1$ is $C_{1-6}$ alkylene.

In some embodiments of Formula (A), Formula (I) and relevant subembodiments thereof, $R^a$ and $R^b$ together with the nitrogen to which they are attached combine to form a structure selected from the group consisting of

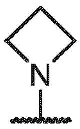, 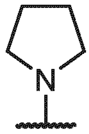, 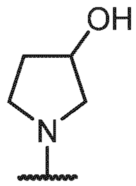, 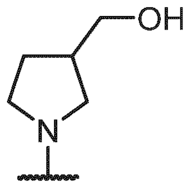, and 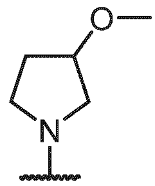.

In some embodiments of Formula (A), Formula (I) and relevant subembodiments thereof, each $R^3$ is independently selected from the group consisting of cyano, $C_1$ haloalkoxy, $C_1$ haloalkyl, -OH, chloro, bromo, and methyl. In some embodiments of Formula (A), Formula (I) and relevant subembodiments thereof, each $R^3$ is independently selected from the group consisting of chloro, bromo, and methyl.

In some embodiments of Formula (A), Formula (I) and relevant subembodiments thereof, each $R^3$ is cyano. In some embodiments of Formula (A), Formula (I) and relevant subembodiments thereof, each $R^3$ is $C_1$ haloalkoxy. In some embodiments of Formula (A), Formula (I) and relevant subembodiments thereof, each $R^3$ is $C_1$ haloalkyl. In some embodiments of Formula (A), Formula (I) and relevant subembodiments thereof, each $R^3$ is - OH.

In some embodiments of Formula (A), Formula (I) and relevant subembodiments thereof, each $R^3$ is chloro. In some embodiments of Formula (A), Formula (I) and relevant subembodiments thereof, $R^3$ is bromo. In some embodiments of Formula (A), Formula (I) and relevant subembodiments thereof, $R^3$ is methyl.

In some embodiments of Formula (A), Formula (I) and relevant subembodiments thereof, the subscript n is 1. In some embodiments of Formula (A), Formula (I) and relevant subembodiments thereof, the subscript n is 0.

In some embodiments of Formula (A), Formula (I) and relevant subembodiments thereof, the compound is selected from a compound in Table 1 or Table 2 or a pharmaceutically acceptable salt thereof.

Representative compounds are listed in Table 1 below:

**Table 1**

| Cpd. No. | Structure | $^1$H NMR | Mass Spec. |
|---|---|---|---|
| 1.001 | 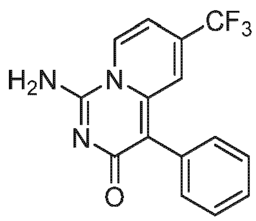 | $^1$H NMR (400 MHz, DMSO-$d_6$) δ 8.02 (d, $J$ = 7.9 Hz, 1H), 7.67 (s, 2H), 7.44 (t, $J$ = 7.5 Hz, 2H), 7.37 (d, $J$ = 7.3 Hz, 1H), 7.27 (d, $J$ = 7.5 Hz, 2H), 6.97 (s, 1H), 6.65 (d, $J$ = 8.1 Hz, 1H). | 306 |
| 1.002 | 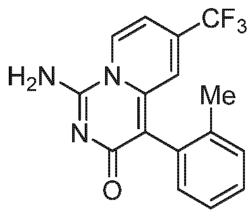 | $^1$H NMR (400 MHz, DMSO-$d_6$) δ 8.03 (d, $J$ = 7.0 Hz, 1H), 7.66 (s, 2H), 7.30 (s, 2H), 7.27 - 7.20 (m, 1H), 7.06 (d, $J$ = 8.6 Hz, 1H), 6.68 - 6.59 (m, 2H), 2.04 (s, 3H). | 320 |
| 1.003 | 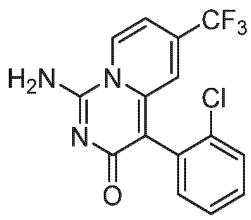 | $^1$H NMR (400 MHz, DMSO-$d_6$) δ 8.08 (d, $J$ = 6.7 Hz, 1H), 7.76 (s, 2H), 7.57 (d, $J$ = 4.7 Hz, 1H), 7.42 (d, $J$ = 4.9 Hz, 2H), 7.30 (d, $J$ = 1.0 Hz, 1H), 6.71 (d, $J$ = 8.1 Hz, 1H), 6.59 (s, 1H) | 340 |
| 1.004 | 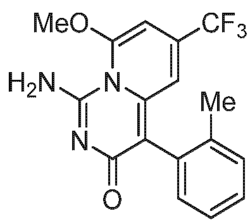 | $^1$H NMR (400 MHz, Chloroform-$d$) δ 7.34 (s, 2H), 7.31 (s, 2H), 7.05 (s, 1H), 6.98 (s, 1H), 5.49 (s, 1H), 5.39 (s, 1H), 4.12 (s, 3H), 2.50 (s, 3H). | 350 |

(continued)

| Cpd. No. | Structure | $^1$H NMR | Mass Spec. |
|---|---|---|---|
| **1.005** | 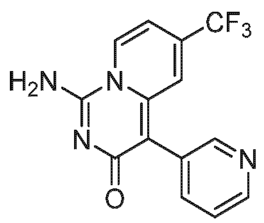 | $^1$HNMR (500 MHz, DMSO-$d_6$) δ 8.54 (dd, J = 5.0, 1.5 Hz, 1H), 8.47 (d, J = 1.5 Hz, 1H), 8.06 (d, J = 7.5 Hz, 1H), 7.80-7.70 (m, 3H), 7.46 (dd, J = 7.5, 5.0 Hz, 1H), 6.97 (s, 1H), 6.70 (dd, J = 7.5, 1.5 Hz, 1H). | 307 |
| **1.006** | 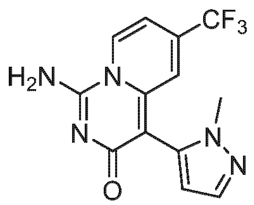 | $^1$HNMR (400 MHz, DMSO-$d_6$) δ 8.10 (d, J = 8.0 Hz, 1H), 7.81 (br s, 2H), 7.47 (d, J = 2.0 Hz, 1H), 7.06 (s, 1H), 6.76 (d, J = 8.0 Hz, 1H), 6.25 (d, J = 2.0 Hz, 1H). | 310 |
| **1.007** | 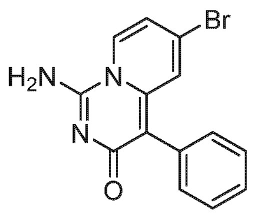 | $^1$HNMR (400 MHz, DMSO-d6) δ 7.85 (d, J = 5.6 Hz, 1H), 7.60-7.39 (m, 4H), 7.36-7.31 (m, 1H), 7.26-7.21 (m, 2H), 6.83 (d, J = 2.0 Hz, 1H), 6.61 (dd, J = 8.0, 2.0 Hz, 1H). | 316 |
| **1.008** | 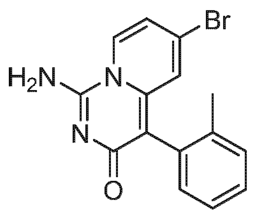 | $^1$HNMR (400 MHz, DMSO-d6) δ 7.84 (d, J = 7.6 Hz, 1H), 7.53 (s, 2H), 7.32-7.20 (m, 3H), 7.16-7.13 (m, 1H), 6.61 (dd, J = 7.6, 2.0 Hz, 1H), 6.49 (d, J = 2.0 Hz, 1H), 2.04 (s, 3H). | 330 |
| **1.009** | 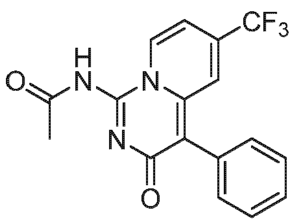 | $^1$HNMR (400 MHz, DMSO-d6) δ 13.3 (br s, 1H), 8.85-8.80 (m, 1H), 7.50-7.45 (m, 3H), 7.38-7.33 (m, 2H), 7.08-7.04 (m, 1H), 6.79 (s, 1H), 2.25 (s, 3H). | 348 |
| **1.010** | 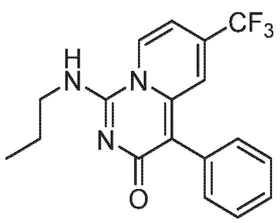 | $^1$HNMR (400 MHz, DMSO-d6) δ 8.08 (d, J = 8.0 Hz, 1H), 7.91 (s, 1H), 7.46-7.41 (m, 2H), 7.38-7.35 (m, 1H), 7.30-7.25 (m, 2H), 6.96 (d, J = 2.0 Hz, 1H), 6.65 (dd, J = 8.0, 2.0 Hz, 1H), 3.38-3.30 (m, 2H), 1.70-1.58 (m, 2H), 0.94 (t, J = 7.2 Hz, 3H). | 348 |
| **1.011** | 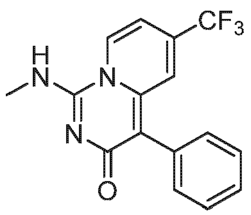 | $^1$HNMR (400 MHz, DMSO-d6) δ 8.05 (d, J = 7.6 Hz, 1H), 8.02-7.90 (br s, 1H), 7.46-7.41 (m, 2H), 7.38-7.35 (m, 1H), 7.30-7.25 (m, 2H), 6.96 (d, J = 1.6 Hz, 1H), 6.63 (dd, J = 7.6, 1.6 Hz, 1H), 2.90 (s, 3H). | 320 |

Additional representative compounds are listed in Table 2 below:

Table 2

| Cpd. No. | Structure |
|---|---|
| 2.001 | 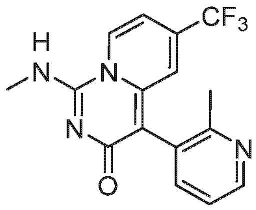 |
| 2.002 | 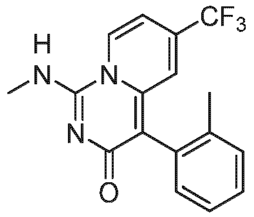 |
| 2.003 | 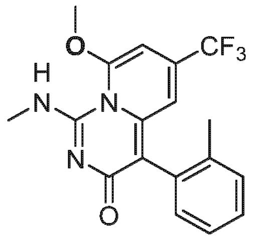 |
| 2.004 | 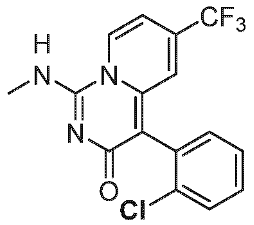 |
| 2.005 | 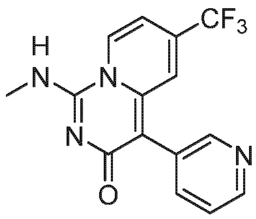 |
| 2.006 | 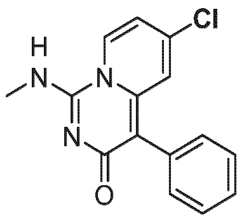 |
| 2.007 | 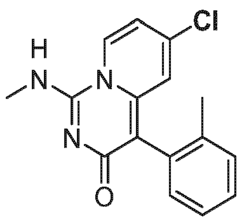 |

(continued)

| Cpd. No. | **Structure** |
|---|---|
| **2.008** | 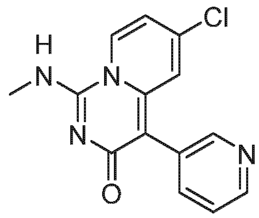 |
| **2.009** | 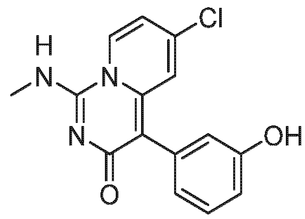 |
| **2.010** | 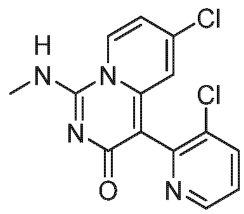 |
| **2.011** | 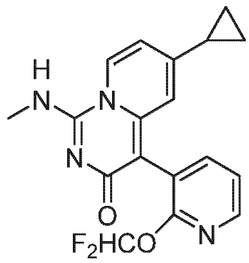 |
| **2.012** | 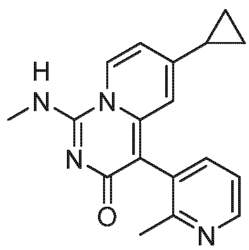 |
| **2.013** | 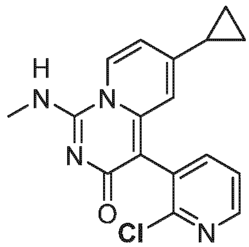 |

(continued)

| Cpd. No. | **Structure** |
|---|---|
| **2.014** | 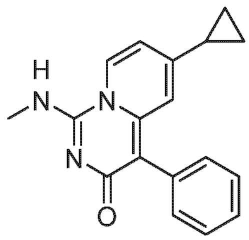 |
| **2.015** | 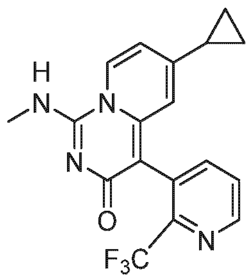 |
| **2.016** | 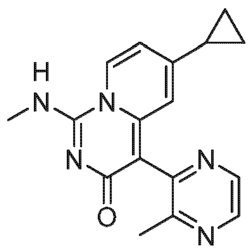 |
| **2.017** | 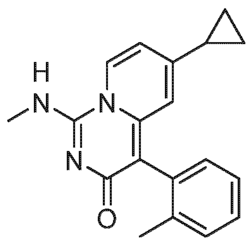 |
| **2.018** | 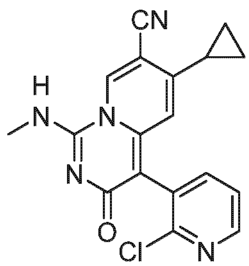 |
| **2.019** | 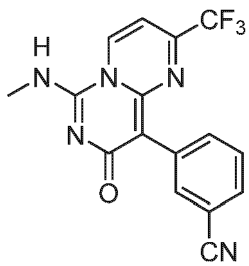 |

(continued)

| Cpd. No. | **Structure** |
|---|---|
| **2.020** | 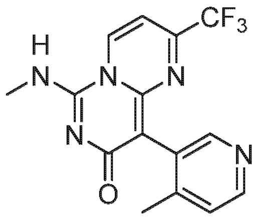 |
| **2.021** | 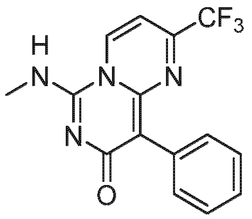 |
| **2.022** | 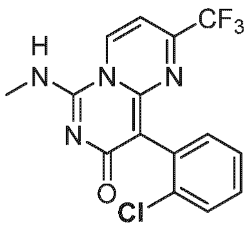 |
| **2.023** | 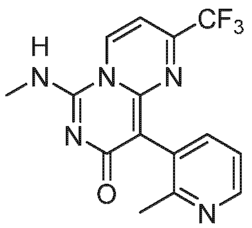 |
| **2.024** | 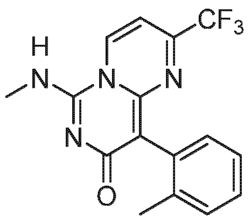 |
| **2.025** | 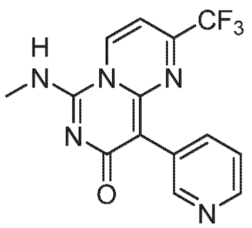 |
| **2.026** | 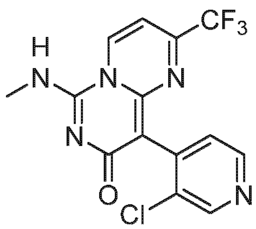 |

(continued)

| Cpd. No. | Structure |
|---|---|
| **2.027** | 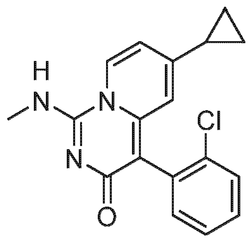 |

**General Synthesis**

Compounds of this disclosure can be made by the methods depicted in the reaction schemes shown below.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this disclosure can be synthesized, and various modifications to these schemes can be made and will be suggested to one skilled in the art reading this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure over a temperature range from about -78 °C to about 150 °C, such as from about 0 °C to about 125 °C and further such as at about room (or ambient) temperature, e.g., about 20°C.

Compounds of Formula (I) and the subembodiments described herein where $R^1$,$R^2$ and $R^3$ are as defined in the Summary can be prepared by the method illustrated and described in Scheme 1 below

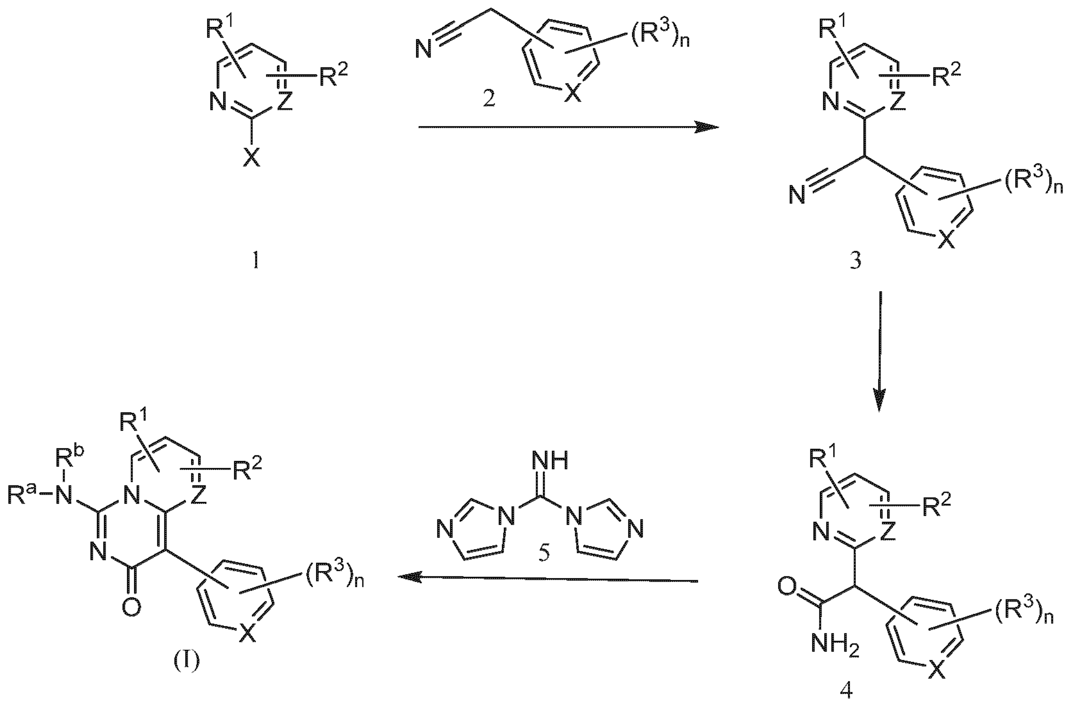

Compounds of formula 1 and 2, where X is halo such as chloro and $R^1$, $R^2$ and $R^3$ are described in Summary or a

precursor group thereof are commercially available. These compounds can be readily converted to a compound of Formula 3 by methods well known in the art. For example, treatment of compound 2 with KOH in in an inert organic solvent followed by addition of 1 provides compound of Formula (3) where the cyano can then be converted to compounds of Formula (4) by adding concentrated acid like $H_2SO_4$. Compounds of Formula (4) can be converted to a compound of Formula (I) using known techniques in the art. In the above diagram, a base such NaH and commercially available reagent 5 are used.

Alternatively, compounds of formula (I) where $R^a$ and $R^b$ are as defined in the Summary can be prepared by the method illustrated and described in Scheme 2 and 3 below.

**Scheme 2**

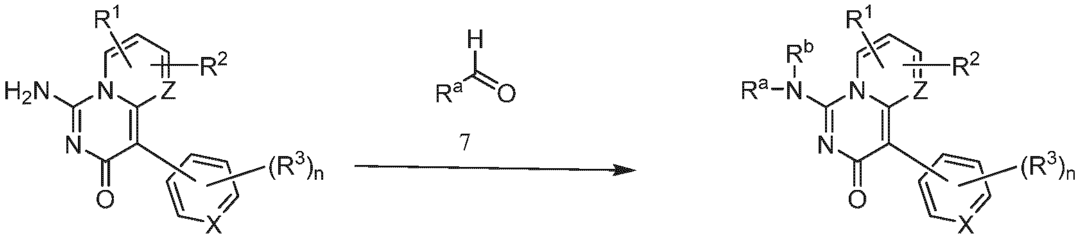

6

7

(I)

Compounds of formula 6 can be prepared using Scheme 1, and 7, where $R^a$, $R^b$, $R^1$, $R^2$ and $R^3$ are described in Summary or a precursor group thereof are commercially available. These compounds can be readily converted to a compound of Formula (I) by methods well known in the art. For example, treatment of compound 6 with AcOH in protic organic solvent followed by addition of 7 and a reducing agent provides compound of Formula (I) where $R^b$ can then be added to compounds of Formula (I) the same way. Non limiting examples of reducing agents are 2-Picoline-borane and sodium borohydride.

**Scheme 3**

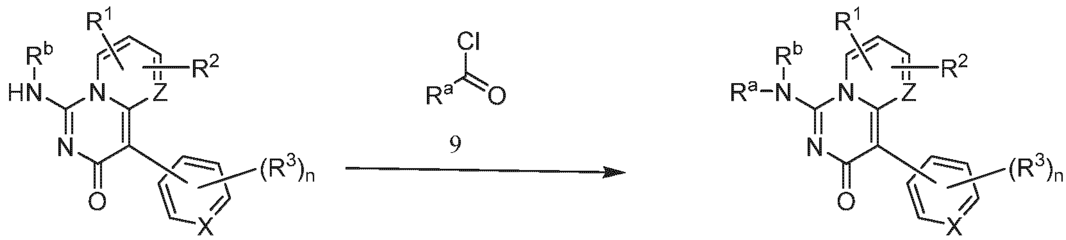

8

9

(I)

Compounds of formula 8 and 9, where $R^a$, $R^b$, $R^1$, $R^2$ and $R^3$ are described in Summary or a precursor group thereof are commercially available. These compounds can be readily converted to a compound of Formula (I) by methods well known in the art. For example, treatment of compound 8 with TEA in aprotic organic solvent followed by addition of 9 provides compound of Formula (I).

It is understood that replacement of the six membered cyclic moiety comrpsing ring vertex "X" with a five or six membered heteroaryl comprising 1 to 3 heteroatom ring vertices independently selected from the group consisting of N, O, and S can provide compounds of Formula (A).

**Utility**

Overexpression of the enzyme MAT2A has been demonstrated to mediate certain cancers. In an embodiment, the cancer is neuroblastoma, intestine carcinoma (such as rectum carcinoma, colon carcinoma, familiary adenomatous polyposis carcinoma and hereditary non-polyposis colorectal cancer), esophageal carcinoma, labial carcinoma, larynx carcinoma, hypopharynx carcinoma, tongue carcinoma, salivary gland carcinoma, gastric carcinoma, adenocarcinoma, medullary thyroid carcinoma, papillary thyroid carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors (such as glioblastoma, astrocytoma, meningioma, medulloblastoma and peripheral neuroectodermal tumors), Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia, hepatocellular carcinoma, gall

bladder carcinoma, bronchial carcinoma, small cell lung carcinoma, non-small cell lung carcinoma, multiple myeloma, basalioma, teratoma, retinoblastoma, choroidea melanoma, seminoma, rhabdomyo sarcoma, craniopharyngeoma, osteosarcoma, chondrosarcoma, myosarcoma, liposarcoma, fibrosarcoma, Ewing sarcoma and plasmocytoma.

In another embodiment, the cancer is lung cancer, non-small cell lung (NSLC) cancer, bronchioloalveolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the vagina, carcinoma of the vulva, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, chronic or acute leukemia, lymphocytic lymphomas, neoplasms of the central nervous system (CNS), spinal axis tumors, brain stem glioma, glioblastoma multiforme, astrocytomas, schwannomas, ependymomas, medulloblastomas, meningiomas, squamous cell carcinomas, pituitary adenomas, including refractory versions of any of the above cancers, or a combination of one or more of the above cancers.

Methylthioadenosine phosphorylase (MTAP) is an enzyme found in all normal tissues that catalyzes the conversion of methylthioadenosine (MTA) into adenine and 5-methylthio-ribose-1-phosphate. The adenine is salvaged to generate adenosine monophosphate, and the 5-methylthioribose-1-phosphate is converted to methionine and formate. Because of this salvage pathway, MTA can serve as an alternative purine source when de novo purine synthesis is blocked, e.g., with antimetabolites, such as L-alanosine.

Many human and murine malignant cells lack MTAP activity. MTAP deficiency is not only found in tissue culture cells but the deficiency is also present in primary leukemias, gliomas, melanomas, pancreatic cancers, non-small cell lung cancers (NSLC), bladder cancers, astrocytomas, osteosarcomas, head and neck cancers, myxoid chondrosarcomas, ovarian cancers, endometrial cancers, breast cancers, soft tissue sarcomas, non-Hodgkin lymphomas, and mesotheliomas. It has been reported by K. Marjon et al., Cell Reports 15 (2016) 574- 587, that proliferation of cancer cells that are MTAP null is inhibited by knocking down MAT2A expression with shRNA. An MTAP null cancer is a cancer in which the MTAP gene has been deleted or lost or otherwise deactivated or a cancer in which the MTAP protein has a reduced or impaired function.

Accordingly, in an embodiment of the present disclosure there is provided a method for treating an MTAP null cancer in a patient wherein said cancer is characterized by a reduction or absence of MTAP expression or absence of the MTAP gene or reduced function of MTAP protein as compared to cancers where the MTAP gene is present and fully functioning, said method comprising administering to the patient in need thereof a therapeutically effective amount of a compound of Formula (A), Formula (I) or a subembodiment described herein or a pharmaceutically acceptable salt thereof. In an embodiment of the present disclosure there is provided a method for treating an MTAP null cancer in a patient wherein said cancer is characterized by a reduction or absence of MTAP expression or absence of the MTAP gene, reduced level of MTAP protein, or reduced function of MTAP protein as compared to cancers where the MTAP gene is present and fully functioning, said method comprising administering to the patient in need thereof a therapeutically effective amount of a compound of Formula (A), Formula (I) or a subembodiment described herein or a pharmaceutically acceptable salt thereof. In another embodiment, provided is a method of treating an MTAP null cancer in a patient comprising administering to the patient in need thereof an effective amount of a compound of Formula (A), Formula (I) or a subembodiment described herein or a pharmaceutically acceptable salt thereof. In an embodiment, the MTAP null cancer is leukemia, glioma, melanoma, pancreatic, non-small cell lung cancer (NSLC), bladder cancer, astrocytoma, osteosarcoma, head and neck cancer, myxoid chondrosarcoma, ovarian cancer, endometrial cancer, breast cancer, soft tissue sarcoma, non-Hodgkin lymphoma or mesothelioma. In another embodiment, the MTAP null cancer is pancreatic cancer. In yet another embodiment, the MTAP null cancer is bladder cancer, melanoma, brain cancer, lung cancer, pancreatic cancer, breast cancer, esophageal cancer, head and neck cancer, kidney cancer, colon cancer, diffuse large B cell lymphoma (DLBCL), acute lymphoblastic leukemia (ALL) or mantle cell lymphoma (MCL). In yet another embodiment, the MTAP null cancer is gastric cancer. In yet another embodiment, the cancer is colon cancer. In yet another embodiment, the MTAP null cancer is liver cancer. In yet another embodiment, the MTAP null cancer is glioblastoma multiforme (GBM). In yet another embodiment, the MTAP null cancer is bladder cancer. In yet another embodiment, the MTAP null cancer is esophageal cancer. In yet another embodiment, the MTAP null cancer is breast cancer. In yet another embodiment, the MTAP null cancer is NSLCC. In yet another embodiment, the MTAP null cancer is MCL. In yet another embodiment, the MTAP null cancer is DLBCL. In yet another embodiment, the MTAP null cancer is ALL.

In another embodiment, the MTAP null cancer is solid tumor. In another embodiment, the MTAP null cancer is malignant solid tumor.

Genomic analysis of MTAP null cell lines has shown that cell lines that also incorporate a KRAS mutation or a p53 mutation were sensitive to MAT2A inhibition.

Accordingly, also provided is a method for treating a cancer in a patient wherein said cancer is characterized by reduction or absence of MTAP expression or absence of the MTAP gene or reduced function of MTAP protein (i..e, MTAP

null) and further characterized by the presence of mutant KRAS and/or mutant p53, said method comprising administering to the patient a therapeutically effective amount of a compound of Formula (A), Formula (I) or a subembodiment described herein. In one embodiment, the cancer is MTAP null and KRAS mutant. In another embodiment, the cancer is MTAP null and p53 mutant. In yet another embodiment, the cancer is MTAP null, KRAS mutant and p53 mutant.

The term "mutant KRAS" or "KRAS mutation" refers to KRAS protein (or gene encoding said protein) incorporating an activating mutation that alters its normal function. For example, a mutant KRAS protein may incorporate a single amino acid substitution at position 12 or 13. In a particular embodiment, the KRAS mutant incorporates a G12X or G13X substitution, wherein X represents any amino acid change at the indicated position. In a particular embodiment, the substitution is G12V, G12R, G12C or G13D. In another embodiment, the substitution is G13D. By "mutant p53" or "p53 mutation" is meant p53 protein (or gene encoding said protein) incorporating a mutation that inhibits or eliminates its tumor suppressor function. In an embodiment, said p53 mutation is, Y126_splice, K132Q, M133K, R174fs, R175H, R196*, C238S, C242Y, G245S, R248W, R248Q, I255T, D259V, S261_splice, R267P, R273C, R282W, A159V or R280K. In an embodiment, the foregoing cancer is non-small cell lung cancer (NSLCC), pancreatic cancer, head and neck cancer, gastric cancer, breast cancer, colon cancer or ovarian cancer.

Assay

The ability of compounds of the disclosure to inhibit MAT2A can be measured as described in Biological Example 1 below.

**Pharmaceutical Composition**

The compounds of Formula (A), Formula (I) or a subembodiment described herein, or a pharmaceutically acceptable salt thereof, may be in the form of compositions suitable for administration to a subject. In general, such compositions are pharmaceutical compositions comprising a compound of Formula (A), Formula (I) or a subembodiment described herein or a pharmaceutically acceptable salt thereof and one or more pharmaceutically acceptable or physiologically acceptable excipients. In certain embodiments, the compound of Formula (A), Formula (I) or a subembodiment described herein, or a pharmaceutically acceptable salt thereof is present in a therapeutically effective amount. The pharmaceutical compositions may be used in the methods disclosed herein; thus, for example, the pharmaceutical compositions can be administered *ex vivo* or *in vivo* to a subject in order to practice the therapeutic methods and uses described herein.

The pharmaceutical compositions can be formulated to be compatible with the intended method or route of administration; exemplary routes of administration are set forth herein. Furthermore, the pharmaceutical compositions may be used in combination with other therapeutically active agents or compounds as described herein in order to treat the diseases, disorders and conditions contemplated by the present disclosure.

The pharmaceutical compositions containing the active ingredient (e.g., a compound of Formula (A), Formula (I) or a subembodiment described herein, a pharmaceutically acceptable salt thereof) may be in a form suitable for oral use, for example, as tablets, capsules, troches, lozenges, aqueous or oily suspensions, dispersible powders or granules, emulsions, hard or soft capsules, or syrups, solutions, microbeads or elixirs. Pharmaceutical compositions intended for oral use may be prepared according to any method known to the art for the manufacture of pharmaceutical compositions, and such compositions may contain one or more agents such as, for example, sweetening agents, flavoring agents, coloring agents and preserving agents in order to provide pharmaceutically elegant and palatable preparations. Tablets, capsules and the like contain the active ingredient in admixture with non-toxic pharmaceutically acceptable excipients which are suitable for the manufacture of tablets, capsules, and the like. These excipients may be, for example, diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc.

The tablets, capsules and the like suitable for oral administration may be uncoated or coated by known techniques to delay disintegration and absorption in the gastrointestinal tract and thereby provide a sustained action. For example, a time-delay material such as glyceryl monostearate or glyceryl di-stearate may be employed. The tablets may also be coated by techniques known in the art to form osmotic therapeutic tablets for controlled release. Additional agents include biodegradable or biocompatible particles or a polymeric substance such as polyesters, polyamine acids, hydrogel, polyvinyl pyrrolidone, polyanhydrides, polyglycolic acid, ethylene-vinyl acetate, methylcellulose, carboxymethylcellulose, protamine sulfate, or lactide and glycolide copolymers, polylactide and glycolide copolymers, or ethylene vinyl acetate copolymers in order to control delivery of an administered composition. For example, the oral agent can be entrapped in microcapsules prepared by coacervation techniques or by interfacial polymerization, by the use of hydroxymethyl cellulose or gelatin-microcapsules or poly (methyl methacrylate) microcapsules, respectively, or in a colloid drug delivery system. Colloidal dispersion systems include macromolecule complexes, nanocapsules, microspheres, microbeads, and lipid-based systems, including oil-in-water emulsions, micelles, mixed micelles, and liposomes. Methods for the preparation of the above-mentioned formulations are known in the art.

Formulations for oral use may also be presented as hard gelatin capsules wherein the active ingredient is mixed with an inert solid diluent, for example, calcium carbonate, calcium phosphate, kaolin or microcrystalline cellulose, or as soft gelatin capsules wherein the active ingredient is mixed with water or an oil medium, for example peanut oil, liquid paraffin, or olive oil.

Aqueous suspensions contain the active materials in admixture with excipients suitable for the manufacture thereof. Such excipients can be suspending agents, for example sodium carboxymethylcellulose, methylcellulose, (hydroxypropyl)methyl cellulose, sodium alginate, polyvinyl-pyrrolidone, gum tragacanth and gum acacia; dispersing or wetting agents, for example a naturally-occurring phosphatide (e.g., lecithin), or condensation products of an alkylene oxide with fatty acids (e.g., poly-oxyethylene stearate), or condensation products of ethylene oxide with long chain aliphatic alcohols (e.g., for heptdecaethyleneoxycetanol), or condensation products of ethylene oxide with partial esters derived from fatty acids and a hexitol (e.g., polyoxyethylene sorbitol monooleate), or condensation products of ethylene oxide with partial esters derived from fatty acids and hexitol anhydrides (e.g., polyethylene sorbitan monooleate). The aqueous suspensions may also contain one or more preservatives.

Oily suspensions may be formulated by suspending the active ingredient in a vegetable oil, for example, arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified herein.

The pharmaceutical compositions may also be in the form of oil-in-water emulsions. The oily phase may be a vegetable oil, for example olive oil or arachis oil, or a mineral oil, for example, liquid paraffin, or mixtures of these. Suitable emulsifying agents may be naturally occurring gums, for example, gum acacia or gum tragacanth; naturally occurring phosphatides, for example, soy bean, lecithin, and esters or partial esters derived from fatty acids; hexitol anhydrides, for example, sorbitan monooleate; and condensation products of partial esters with ethylene oxide, for example, polyoxyethylene sorbitan monooleate.

The pharmaceutical compositions typically comprise a therapeutically effective amount of a compound of Formula (A), Formula (I) or a subembodiment described herein, or a salt thereof, and one or more pharmaceutically acceptable excipient. Suitable pharmaceutically acceptable excipients include, but are not limited to, antioxidants (e.g., ascorbic acid and sodium bisulfate), preservatives (e.g., benzyl alcohol, methyl parabens, ethyl or n-propyl, p-hydroxybenzoate), emulsifying agents, suspending agents, dispersing agents, solvents, fillers, bulking agents, detergents, buffers, vehicles, diluents, and/or adjuvants. For example, a suitable vehicle may be physiological saline solution or citrate buffered saline, possibly supplemented with other materials common in pharmaceutical compositions for parenteral administration. Neutral buffered saline or saline mixed with serum albumin are further exemplary vehicles. Those skilled in the art will readily recognize a variety of buffers that can be used in the pharmaceutical compositions and dosage forms contemplated herein. Typical buffers include, but are not limited to, pharmaceutically acceptable weak acids, weak bases, or mixtures thereof. As an example, the buffer components can be water soluble materials such as phosphoric acid, tartaric acids, lactic acid, succinic acid, citric acid, acetic acid, ascorbic acid, aspartic acid, glutamic acid, and salts thereof. Acceptable buffering agents include, for example, a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), and N-tris[Hydroxymethyl]methyl-3-aminopropanesulfonic acid (TAPS).

After a pharmaceutical composition has been formulated, it may be stored in sterile vials as a solution, suspension, gel, emulsion, solid, or dehydrated or lyophilized powder. Such formulations may be stored either in a ready-to-use form, a lyophilized form requiring reconstitution prior to use, a liquid form requiring dilution prior to use, or other acceptable form. In some embodiments, the pharmaceutical composition is provided in a single-use container (e.g., a single-use vial, ampoule, syringe, or autoinjector (similar to, e.g., an EpiPen®)), whereas a multi-use container (e.g., a multi-use vial) is provided in other embodiments.

Formulations can also include carriers to protect the composition against rapid degradation or elimination from the body, such as a controlled release formulation, including liposomes, hydrogels, prodrugs and microencapsulated delivery systems. For example, a time delay material such as glyceryl monostearate or glyceryl stearate alone, or in combination with a wax, may be employed. Any drug delivery apparatus may be used to deliver a compound of Formula (A), Formula (I) or a subembodiment described herein, or a salt thereof, including implants (e.g., implantable pumps) and catheter systems, slow injection pumps and devices, all of which are well known to the skilled artisan.

Depot injections, which are generally administered subcutaneously or intramuscularly, may also be utilized to release the compound of Formula (A), Formula (I) or a subembodiment described herein, or a salt thereof over a defined period of time. Depot injections are usually either solid- or oil-based and generally comprise at least one of the formulation

components set forth herein. One of ordinary skill in the art is familiar with possible formulations and uses of depot injections.

The pharmaceutical compositions may be in the form of a sterile injectable aqueous or oleagenous suspension. The suspension may be formulated according to the known art using those suitable dispersing or wetting agents and suspending agents mentioned herein. The sterile injectable preparation may also be a sterile injectable solution or suspension in a non-toxic parenterally-acceptable diluent or solvent, for example, as a solution in 1,3-butane diol. Acceptable diluents, solvents and dispersion media that may be employed include water, Ringer's solution, isotonic sodium chloride solution, Cremophor EL™ (BASF, Parsippany, NJ) or phosphate buffered saline (PBS), ethanol, polyol (e.g., glycerol, propylene glycol, and liquid polyethylene glycol), and suitable mixtures thereof. In addition, sterile, fixed oils are conventionally employed as a solvent or suspending medium. For this purpose, any bland fixed oil may be employed, including synthetic mono- or diglycerides. Moreover, fatty acids such as oleic acid, find use in the preparation of injectables. Prolonged absorption of particular injectable formulations can be achieved by including an agent that delays absorption (e.g., aluminum monostearate or gelatin).

A compound of Formula (A), Formula (I) or a subembodiment described herein, or a salt thereof may also be administered in the form of suppositories for rectal administration or sprays for nasal or inhalation use. The suppositories can be prepared by mixing the drug with a suitable non-irritating excipient which is solid at ordinary temperatures but liquid at the rectal temperature and will therefore melt in the rectum to release the drug. Such materials include, but are not limited to, cocoa butter and polyethylene glycols.

## Routes of Administration

Compounds of Formula (A), Formula (I) or a subembodiment described herein, or a salt thereof and compositions containing the same may be administered in any appropriate manner. Suitable routes of administration include oral, parenteral (e.g., intramuscular, intravenous, subcutaneous (e.g., injection or implant), intraperitoneal, intracisternal, intraarticular, intraperitoneal, intracerebral (intraparenchymal) and intracerebroventricular), nasal, vaginal, sublingual, intraocular, rectal, topical (e.g., transdermal), buccal and inhalation. Depot injections, which are generally administered subcutaneously or intramuscularly, may also be utilized to administer the compounds of Formula (A), Formula (I) or a subembodiment described herein, or a salt thereof over a defined period of time. Particular embodiments of the present invention contemplate oral administration.

## Combination Therapy

The present invention contemplates the use of compounds of Formula (A), Formula (I) or a subembodiment described herein, or a salt thereof in combination with one or more active therapeutic agents (e.g., chemotherapeutic agents) or other prophylactic or therapeutic modalities (e.g., radiation). In such combination therapy, the various active agents frequently have different, complementary mechanisms of action. Such combination therapy may be especially advantageous by allowing a dose reduction of one or more of the agents, thereby reducing or eliminating the adverse effects associated with one or more of the agents. Furthermore, such combination therapy may have a synergistic therapeutic or prophylactic effect on the underlying disease, disorder, or condition.

As used herein, "combination" is meant to include therapies that can be administered separately, for example, formulated separately for separate administration (e.g., as may be provided in a kit), and therapies that can be administered together in a single formulation (i.e., a "co-formulation").

In certain embodiments, the compounds of Formula (A), Formula (I) or a subembodiment described herein, or a salt thereof are administered or applied sequentially, e.g., where one agent is administered prior to one or more other agents. In other embodiments, the compounds of Formula (A), Formula (I) or a subembodiment described herein, or a salt thereof are administered simultaneously, e.g., where two or more agents are administered at or about the same time; the two or more agents may be present in two or more separate formulations or combined into a single formulation (i.e., a co-formulation). Regardless of whether the two or more agents are administered sequentially or simultaneously, they are considered to be administered in combination for purposes of the present disclosure.

The compounds of Formula (A), Formula (I) or a subembodiment described herein, or a salt thereof may be used in combination with at least one other (active) agent in any manner appropriate under the circumstances. In one embodiment, treatment with the at least one active agent and at least one compound of Formula (A), Formula (I) or a subembodiment described herein, or a salt thereof is maintained over a period of time. In another embodiment, treatment with the at least one active agent is reduced or discontinued (e.g., when the subject is stable), while treatment with the compound of Formula (A), Formula (I) or a subembodiment described herein, or a salt thereof is maintained at a constant dosing regimen. In a further embodiment, treatment with the at least one active agent is reduced or discontinued (e.g., when the subject is stable), while treatment with a compound of Formula (A), Formula (I) or a subembodiment described herein, or a salt thereof is reduced (e.g., lower dose, less frequent dosing or shorter treatment regimen). In yet another

embodiment, treatment with the at least one active agent is reduced or discontinued (e.g., when the subject is stable), and treatment with the compound of Formula (A), Formula (I) or a subembodiment described herein, or a salt thereof is increased (e.g., higher dose, more frequent dosing or longer treatment regimen). In yet another embodiment, treatment with the at least one active agent is maintained and treatment with the compound of Formula (A), Formula (I) or a subembodiment described herein, or a salt thereof is reduced or discontinued (e.g., lower dose, less frequent dosing or shorter treatment regimen). In yet another embodiment, treatment with the at least one active agent and treatment with the compound of Formula (A), Formula (I) or a subembodiment described herein, or a salt thereof are reduced or discontinued (e.g., lower dose, less frequent dosing or shorter treatment regimen).

The present disclosure provides methods for treating cancer with a compound of Formula (A), Formula (I) or a subembodiment described herein, or a salt thereof and at least one additional therapeutic or diagnostic agent.

In some embodiments, the compound of Formula (A), Formula (I) or a subembodiment described herein, or a salt thereof is administered in combination with at least one additional therapeutic agent, selected from Temozolomide, Pemetrexed, Pegylated liposomal doxorubicin (Doxil), Eribulin (Halaven), Ixabepilone (Ixempra), Protein-bound paclitaxel (Abraxane), Oxaliplatin, Irinotecan, Venatoclax (bcl2 inhibitor), 5-azacytadine, Anti-CD20 therapeutics, such as Rituxan and obinutuzumab, Hormonal agents (anastrozole, exemestand, letrozole, zoladex, lupon eligard), CDK4/6 inhibitors, Palbociclib, Abemaciclib, CPI (Avelumab, Cemiplimab-rwlc, and Bevacizumab.

In certain embodiments, the present disclosure provides methods for treating cancer comprising administration of a compound of Formula (A), Formula (I) or a subembodiment described herein, or a salt thereof in combination with a signal transduction inhibitor (STI) to achieve additive or synergistic suppression of tumor growth. As used herein, the term "signal transduction inhibitor" refers to an agent that selectively inhibits one or more steps in a signaling pathway. Examples of signal transduction inhibitors (STIs) useful in methods described herein include, but are not limited to: (i) bcr/abl kinase inhibitors (e.g., GLEEVEC); (ii) epidermal growth factor (EGF) receptor inhibitors, including kinase inhibitors and antibodies; (iii) her-2/neu receptor inhibitors (e.g., HERCEPTIN); (iv) inhibitors of Akt family kinases or the Akt pathway (e.g., rapamycin); (v) cell cycle kinase inhibitors (e.g., flavopiridol); and (vi) phosphatidyl inositol kinase inhibitors. Agents involved in immunomodulation can also be used in combination with one or more compounds of Formula (A), Formula (I) or a subembodiment described herein, or a salt thereof for the suppression of tumor growth in cancer patients.

In certain embodiments, the present disclosure provides methods for treating cancer comprising administration of a compound of Formula (A), Formula (I) or a subembodiment described herein, or a salt thereof in combination with a chemotherapeutic agents. Examples of chemotherapeutic agents include, but are not limited to, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime; nitrogen mustards such as chiorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside (Ara-C); cyclophosphamide; thiotepa; taxoids, e.g., paclitaxel and doxetaxel; chlorambucil; gemcitabine; 6-thioguanine; mercaptopurine; methotrexate; platinum and platinum coordination complexes such as cisplatin and carboplatin; vinblastine; etoposide (VP-16); ifosfamide; mitomycin C; mitoxantrone; vincristine; vinorelbine; navelbine; novantrone; teniposide; daunomycin; aminopterin; xeloda; ibandronate; CPT11; topoisomerase inhibitors; difluoromethylornithine (DMFO); retinoic acid; esperamicins; capecitabine; and pharmaceutically acceptable salts, acids or derivatives of any of the above. In a particular embodiment, compounds of the present disclosure are coadministered with a cytostatic compound selected from the group consisting of cisplatin, doxorubicin, taxol, taxotere and mitomycin C. In a particular embodiment, the cytostatic compound is doxorubicin.

Chemotherapeutic agents also include anti-hormonal agents that act to regulate or inhibit hormonal action on tumors such as anti-estrogens, including for example tamoxifen, raloxifene, aromatase inhibiting 4(5)-imidazoles, 4-hydroxytamoxifen, trioxifene, keoxifene, onapristone, and toremifene; and antiandrogens such as flutamide, nilutamide, bicalutamide, enzalutamide, apalutamide, abiraterone acetate, leuprolide, and goserelin; and pharmaceutically acceptable salts, acids or derivatives of any of the above. In certain embodiments, combination therapy comprises administration of a hormone or related hormonal agent.

The present disclosure also contemplates the use of the compounds of Formula (A), Formula (I) or a subembodiment described herein, or a salt thereof in combination with immune checkpoint inhibitors. The tremendous number of genetic and epigenetic alterations that are characteristic of all cancers provides a diverse set of antigens that the immune system can use to distinguish tumor cells from their normal counterparts. In the case of T cells, the ultimate amplitude (e.g., levels of cytokine production or proliferation) and quality (e.g., the type of immune response generated, such as the pattern of cytokine production) of the response, which is initiated through antigen recognition by the T-cell receptor (TCR), is regulated by a balance between co-stimulatory and inhibitory signals (immune checkpoints). Under normal physiological conditions, immune checkpoints are crucial for the prevention of autoimmunity (i.e., the maintenance of self-tolerance) and also for the protection of tissues from damage when the immune system is responding to pathogenic infection. The expression of immune checkpoint proteins can be dysregulated by tumors as an important immune resistance mechanism. Examples of immune checkpoint inhibitors include but are not limited to CTLA-4, PD-1, PD-L1, BTLA, TIM3, LAG3, OX40, 41BB, VISTA, CD96, TGFβ, CD73, CD39, A2AR, A2BR, IDO1, TDO2, Arginase, B7-H3, B7-H4. Cell-based modulators of anti-cancer immunity are also contemplated. Examples of such modulators include but are not limited to chimeric antigen receptor T-cells, tumor infiltrating T-cells and dendritic-cells.

The present disclosure contemplates the use of compounds of Formula (A), Formula (I) or a subembodiment described herein, or a salt thereof in combination with inhibitors of the aforementioned immune-checkpoint receptors and ligands, for example ipilimumab, abatacept, nivolumab, pembrolizumab, atezolizumab, nivolumab, and durvalumab.

Additional treatment modalities that may be used in combination with a compound of Formula (A), Formula (I) or a subembodiment described herein, or a salt thereof include radiotherapy, a monoclonal antibody against a tumor antigen, a complex of a monoclonal antibody and toxin, a T-cell adjuvant, bone marrow transplant, or antigen presenting cells (e.g., dendritic cell therapy).

The present disclosure contemplates the use of compounds of Formula (A), Formula (I) or a subembodiment described herein, or a salt thereof for the treatment of glioblastoma either alone or in combination with radiation and/or temozolomide (TMZ), avastin or lomustine.

The present disclosure encompasses pharmaceutically acceptable salts, acids or derivatives of any of the above.

## Dosing

The compounds of Formula (A), Formula (I) or a subembodiment described herein, or a salt thereof may be administered to a subject in an amount that is dependent upon, for example, the goal of administration (e.g., the degree of resolution desired); the age, weight, sex, and health and physical condition of the subject to which the formulation is being administered; the route of administration; and the nature of the disease, disorder, condition or symptom thereof. The dosing regimen may also take into consideration the existence, nature, and extent of any adverse effects associated with the agent(s) being administered. Effective dosage amounts and dosage regimens can readily be determined from, for example, safety and dose-escalation trials, in vivo studies (e.g., animal models), and other methods known to the skilled artisan.

In general, dosing parameters dictate that the dosage amount be less than an amount that could be irreversibly toxic to the subject (the maximum tolerated dose (MTD)) and not less than an amount required to produce a measurable effect on the subject. Such amounts are determined by, for example, the pharmacokinetic and pharmacodynamic parameters associated with ADME, taking into consideration the route of administration and other factors.

An effective dose (ED) is the dose or amount of an agent that produces a therapeutic response or desired effect in some fraction of the subjects taking it. The "median effective dose" or $ED_{50}$ of an agent is the dose or amount of an agent that produces a therapeutic response or desired effect in 50% of the population to which it is administered. Although the $ED_{50}$ is commonly used as a measure of reasonable expectance of an agent's effect, it is not necessarily the dose that a clinician might deem appropriate taking into consideration all relevant factors. Thus, in some situations the effective amount is more than the calculated $ED_{50}$, in other situations the effective amount is less than the calculated $ED_{50}$, and in still other situations the effective amount is the same as the calculated $ED_{50}$.

In addition, an effective dose of a compound of Formula (A), Formula (I) or a subembodiment described herein, or a salt thereof may be an amount that, when administered in one or more doses to a subject, produces a desired result relative to a healthy subject. For example, for a subject experiencing a particular disorder, an effective dose may be one that improves a diagnostic parameter, measure, marker and the like of that disorder by at least about 5%, at least about

10%, at least about 20%, at least about 25%, at least about 30%, at least about 40%, at least about 50%, at least about 60%, at least about 70%, at least about 80%, at least about 90%, or more than 90%, where 100% is defined as the diagnostic parameter, measure, marker and the like exhibited by a normal subject.

In certain embodiments, the compounds of Formula (A), Formula (I) or a subembodiment described herein, or a salt thereof may be administered (e.g., orally) at dosage levels of about 0.01 mg/kg to about 50 mg/kg, or about 1 mg/kg to about 25 mg/kg, of subject body weight per day, one or more times a day, to obtain the desired therapeutic effect.

For administration of an oral agent, the compositions can be provided in the form of tablets, capsules and the like containing from 1.0 to 1000 milligrams of the active ingredient, particularly 1.0, 3.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient.

In certain embodiments, the dosage of the compound of Formula (A), Formula (I) or a subembodiment described herein, or a salt thereof is contained in a "unit dosage form". The phrase "unit dosage form" refers to physically discrete units, each unit containing a predetermined amount of the compound of Formula (A), Formula (I) or a subembodiment described herein, or a salt thereof, either alone or in combination with one or more additional agents, sufficient to produce the desired effect. It will be appreciated that the parameters of a unit dosage form will depend on the particular agent and the effect to be achieved.

**Kits**

The present description also contemplates kits comprising a compound of Formula (A), Formula (I) or a subembodiment described herein, or a salt thereof, and pharmaceutical compositions thereof. Kits are not part of the invention. The kits are generally in the form of a physical structure housing various components, as described below, and may be utilized, for example, in practicing the methods described above.

A kit can include one or more of the compound of Formula (A), Formula (I) or a subembodiment described herein, or a salt thereof (provided in, e.g., a sterile container), which may be in the form of a pharmaceutical composition suitable for administration to a subject. The compound of Formula (A), Formula (I) or a subembodiment described herein, or a salt thereof can be provided in a form that is ready for use (e.g., a tablet or capsule) or in a form requiring, for example, reconstitution or dilution (e.g., a powder) prior to administration. When the compounds of Formula (A), Formula (I) or a subembodiment described herein, or a salt thereof are in a form that needs to be reconstituted or diluted by a user, the kit may also include diluents (e.g., sterile water), buffers, pharmaceutically acceptable excipients, and the like, packaged with or separately from the compounds of Formula (A), Formula (I) or a subembodiment described herein, for a salt thereof. When combination therapy is contemplated, the kit may contain the several agents separately or they may already be combined in the kit. Each component of the kit may be enclosed within an individual container, and all of the various containers may be within a single package. A kit of the present invention may be designed for conditions necessary to properly maintain the components housed therein (e.g., refrigeration or freezing).

A kit may contain a label or packaging insert including identifying information for the components therein and instructions for their use (e.g., dosing parameters, clinical pharmacology of the active ingredient(s), including mechanism of action, pharmacokinetics and pharmacodynamics, adverse effects, contraindications, etc.). Labels or inserts can include manufacturer information such as lot numbers and expiration dates. The label or packaging insert may be, e.g., integrated into the physical structure housing the components, contained separately within the physical structure, or affixed to a component of the kit (e.g., an ampule, tube or vial).

Labels or inserts can additionally include, or be incorporated into, a computer readable medium, such as a disk (e.g., hard disk, card, memory disk), optical disk such as CD- or DVD-ROM/RAM, DVD, MP3, magnetic tape, or an electrical storage media such as RAM and ROM or hybrids of these such as magnetic/optical storage media, FLASH media or memory-type cards. In some embodiments, the actual instructions are not present in the kit, but means for obtaining the instructions from a remote source, e.g., via the internet, are provided.

**EXAMPLES**

The following examples and references (intermediates) are put forth so as to provide those of ordinary skill in the art with a complete disclosure and description of how to make and use the present invention, and are not intended to limit the scope of the appendant claims, nor are they intended to represent that the experiments below were performed or that they are all of the experiments that may be performed. It is to be understood that exemplary descriptions written in the present tense were not necessarily performed, but rather that the descriptions can be performed to generate data and the like of a nature described therein. Efforts have been made to ensure accuracy with respect to numbers used (e.g., amounts, temperature, etc.), but some experimental errors and deviations should be accounted for.

Unless indicated otherwise, parts are parts by weight, molecular weight is weight average molecular weight, temperature is in degrees Celsius (°C), and pressure is at or near atmospheric. Standard abbreviations are used, including the following: μg = microgram; μl or μL = microliter; mM = millimolar; μM = micromolar; aa = amino acid(s); $Ac_2O$ = acetic

anhydride; AcCl = acetylchloride;ACN = acetonitrile; AIBN = 2,2'-Azobis(2-methylpropionitrile); BID = twice daily; BINAP = 2,2'-bis(diphenylphosphino)-1 ,1 '-binaphthyl; $Boc_2O$ or $(Boc)_2O$ = di-tert-butyl dicarbonate; bp = base pair(s); BSA = bovine serum albumin; BW = body weight; d = doublet; dd = doublet of doublets; DEAD = diethyl azodicarboxylate; DIBAL = diisobutylaluminium hydride DIEA = N,N-diisopropylethylamine; DIPEA = N,N-diisopropylethylamine; dl or dL = deciliter; DMA = dimethylacetamide; DMAP = dimethylaminopyridine; DME = 1,2-dimethoxyethane; DMEM = Dulbeco's Modification of Eagle's Medium; DMF = N,N-dimethylformamide; DMSO = dimethylsulfoxide; dppf = 1,1'-Bis(diphenylphosphino) ferrocene; DTT = dithiothreitol; EDTA = ethylenediaminetetraacetic acid; ES = electrospray; EtOAc = ethyl acetate; EtOH = ethanol; g = gram; h or hr = hour(s); HATU = 2-(1 H-7-azabenzotriazol-1-yl)-1,1,3,3-tetramethyluronium hexafluorophosphate; HEPES = 4-(2-hydroxyethyl)-1-piperazineethylanesulfonic acid; HOAc = acetic acid; HPLC = high performance liquid chromatography; HPLC = high pressure liquid chromatography; i.m. = intramuscular(ly); i.p. = intraperitoneal(ly); IHC = immunohistochemistry; IPA = isopropyl alcohol; kb = kilobase(s); kDa = kilodalton; kg = kilogram; l or L = liter; LC = liquid chromatography; LCMS = liquid chromatography and mass spectrometry; m / z = mass to charge ratio; M = molar; m = multiplet; MeCN = acetonitrile; MeOH = methanol; $MeSO_2Cl$ = methanesulfonylchloride; mg = milligram; min = minute(s); min = minutes; ml or mL = milliliter; mM = millimolar; MS = mass spectrometry; MsCl = methanesulfonylchloride; N = normal; NADPH = nicotinamide adenine dinucleotide phosphate; NBS = N-bromosuccinamide; ng = nanogram; nm = nanometer; nM = nanomolar; NMP = N-methylpyrrolidone; NMR = nuclear magnetic resonance; ns = not statistically significant; nt = nucleotides(s); PBS = phosphate-buffered saline; Pd/C = palladium on carbon; $Pd_2(dba)_3$ = Tris(debenzylideneactone) dipalladium;$Pd(dppf)Cl_2$ = 1,1'-bis(diphenylphosphino)ferrocene-palladium(II)dichloride; PE = petroleum ether; QD = daily; QM = monthly; QW = weekly; rac = racemic; Rt = retention time; s = singlet; s or sec = second(s); sat. = saturated; SC or SQ = subcutaneous(ly); t = triplet; TBAB = tetra-n-butylammonium bromide; TEA = triethylamine; TFA = trifluoroacetic acid; THF = tetrahydrofuran; TLC = thin layer chromatography; TMSCl = trimethylsilylchloride; TsOH = p-toluenesulfonic acid; U = unit; wt = wildtype.

**Synthetic Examples**

**Example 1:** Synthesis of 1-amino-4-phenyl-6-(trifluoromethyl)-3H-pyrido[1,2-c]pyrimidin-3-one

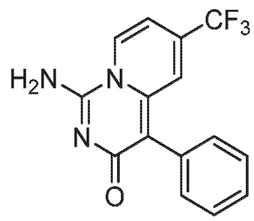

Step 1: Preparation of 2-phenyl-2-(4-(trifluoromethyl)pyridin-2-yl)acetonitrile

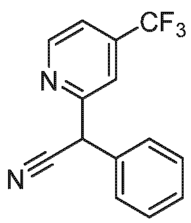

To DMSO (2.5 mL) was added powdered potassium hydroxide (1.1 g, 20.3 mmol, 3.00 equiv) and the resulting mixture was stirred at RT for 30 min. 2-phenylacetonitrile (0.87 g, 7.43 mmol, 1.10 equiv) was added dropwise and the mixture stirred at RT for 30 min followed by addition 2-chloro-4-(trifluoromethyl)pyridine (1.2 g, 6.76 mmol, 1.00 equiv) and stirring at RT for 1 hr. The reaction was quenched by addition of 25 mL water and neutralized with aqueous 1M HCl and the mixture was extracted with DCM (3 x 50 mL). The organic layer was combined, washed with brine, dried over anhydrous $Na_2SO_4$, filtered and concentrated to afford 2-phenyl-2-(4-(trifluoromethyl)pyridin-2-yl)acetonitrile as a white solid. LC-MS (ESI, m/z): 263 [M+H]+.

Step 2: Preparation of 2-phenyl-2-(4-(trifluoromethyl)pyridin-2-yl)acetamide

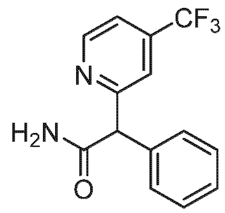

To conc. $H_2SO_4$ (2.5 mL) at 0 °C was added dropwise 2-phenyl-2-(4-(trifluoromethyl)pyridin-2-yl)acetonitrile (1.8 g, 6.76 mmol, 1.00 equiv) and the resulting mixture was stirred at RT for 2 h. The reaction was quenched by addition of 50 mL water and adjusted to pH 5-6 with $NaHCO_3$. The resulting solid was collected by filtration to afford 2-phenyl-2-(4-(trifluoromethyl)pyridin-2-yl)acetamide as a white solid. LC-MS (ESI, m/z): 281 [M+H]+.

Step 3: Preparation of 1-amino-4-phenyl-6-(trifluoromethyl)-3H-pyrido[1,2-c]pyrimidin-3-one

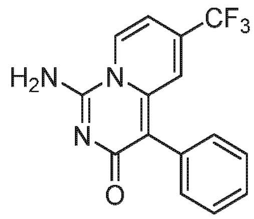

To a solution of 2-phenyl-2-(4-(trifluoromethyl)pyridin-2-yl)acetamide (0.19 g, 0.40 mmol, 1.00 equiv) in DMF (1 mL) was added NaH 60% in mineral oil (0.06 g, 1.62 mmol, 4 equiv) and the resulting mixture stirred at RT for 10 min. Di(1H-imidazol-1-yl)methanimine (0.14 g, 0.89 mmol, 2.2 equiv) was added and the mixture stirred at RT for 2 h. The solvent was removed under reduced pressure and the product purified by Prep-HPLC (20-80% ACN/water, with 0.1% Formic acid) to afford 1-amino-4-phenyl-6-(trifluoromethyl)-3H-pyrido[1,2-c]pyrimidin-3-one as a white solid. $^1H$ NMR (400 MHz, DMSO-$d_6$) $\delta$ 8.02 (d, $J$ = 7.9 Hz, 1H), 7.67 (s, 2H), 7.44 (t, $J$ = 7.5 Hz, 2H), 7.37 (d, $J$ = 7.3 Hz, 1H), 7.27 (d, $J$ = 7.5 Hz, 2H), 6.97 (s, 1H), 6.65 (d, $J$ = 8.1 Hz, 1H). LC-MS (ESI, m/z): 306 [M+H]+.

**Example 2:** Synthesis of 1-amino-4-(o-tolyl)-6-(trifluoromethyl)-3H-pyrido[1,2-c]pyrimidin-3-one

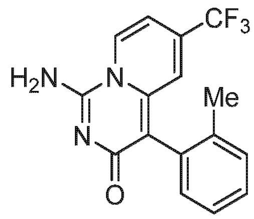

The title compound was synthesized following the method shown in example 1 by substituting 2-(o-tolyl) acetonitrile for 2-phenylacetonitrile in step 1. $^1H$ NMR (400 MHz, DMSO-$d_6$) $\delta$ 8.03 (d, $J$ = 7.0 Hz, 1H), 7.66 (s, 2H), 7.30 (s, 2H), 7.27 - 7.20 (m, 1H), 7.06 (d, $J$ = 8.6 Hz, 1H), 6.68 - 6.59 (m, 2H), 2.04 (s, 3H). LC-MS (ESI, m/z): 320 [M+H]+.

**Example 3:** Synthesis of 1-amino-4-(2-chlorophenyl)-6-(trifluoromethyl)-3H-pyrido[1,2-c]pyrimidin-3-one

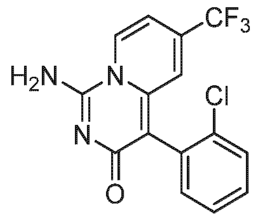

The title compound was synthesized following the method shown in example 1 by substituting 2-(2-chlorophenyl) acetonitrile for 2-phenylacetonitrile in step 1. $^1$H NMR (400 MHz, DMSO-$d_6$) δ 8.08 (d, $J$ = 6.7 Hz, 1H), 7.76 (s, 2H), 7.57 (d, $J$ = 4.7 Hz, 1H), 7.42 (d, $J$ = 4.9 Hz, 2H), 7.30 (d, $J$ = 1.0 Hz, 1H), 6.71 (d, $J$ = 8.1 Hz, 1H), 6.59 (s, 1H). LC-MS (ESI, m/z): 340 [M+H]+.

**Example 4:** Synthesis of 1-amino-8-methoxy-4-(o-tolyl)-6-(trifluoromethyl)-3H-pyrido[1,2-c]pyrimidin-3-one

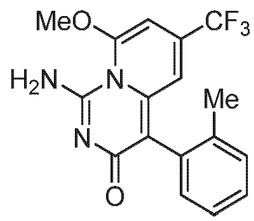

The title compound was synthesized following the method shown in example 1 by substituting 2-(o-tolyl) acetonitrile for 2-phenylacetonitrile and 2-chloro-6-methoxy-4-(trifluoromethyl)pyridine for 2-chloro-4-(trifluoromethyl) pyridine in step 1. $^1$H NMR (400 MHz, Chloroform-$d$) δ 7.34 (s, 2H), 7.31 (s, 2H), 7.05 (s, 1H), 6.98 (s, 1H), 5.49 (s, 1H), 5.39 (s, 1H), 4.12 (s, 3H), 2.50 (s, 3H). LC-MS (ESI, m/z): 350 [M+H]+.

**Example 5:** Synthesis of -Amino-4-(pyridin-3-yl)-6-(trifluoromethyl)-3*H*-pyrido[1,2-c]pyrimidin-3-one

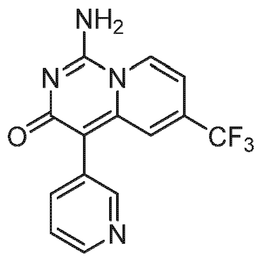

Step 1: Preparation of 2-(Pyridin-3-yl)-2-(4-(trifluoromethyl)pyridin-2-yl)acetonitrile

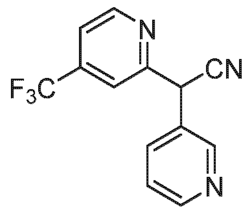

A mixture of KOH (0.924 g, 16.5 mmol, 3.00 equiv) and DMSO (5 mL) was stirred at RT for 30 min. To the above solution was then added 2-(pyridin-3-yl)acetonitrile (0.644 g, 5.5 mmol, 1 equiv) at RT and the resulting solution was stirred for 30 min, followed by addition 2-chloro-4-(trifluoromethyl)pyridine (1 g, 5.5 mmol, 1.00 equiv). After 30 min at RT, water

(20 mL) was added and the mixture was extracted with DCM (3 x 50 mL). The combined organic layers were washed with brine (1 x 20 mL), dried over $Na_2SO_4$, filtered and concentrated to afford 2-(pyridin-3-yl)-2-(4-(trifluoromethyl)pyridin-2-yl) acetonitrile (1 g, 69%) as a pale yellow liquid. LC-MS (ESI, m/z): 264.08 $[M+H]^+$.

Step 2: Preparation of 2-(Pyridin-3-yl)-2-(4-(trifluoromethyl)pyridin-2-yl)acetamide

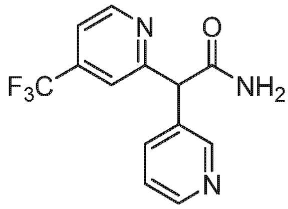

2-(Pyridin-3-yl)-2-(4-(trifluoromethyl)pyridin-2-yl)acetonitrile (1.0 g, 3.8 mmol, 1.00 equiv) was added to concentrated $H_2SO_4$ (5 mL) at 0 °C and the resulting mixture was stirred at RT for 2 h. Water (50 mL) was added and the pH of the reaction mixture was adjusted to 5-6 by addition of saturated aqueous $NaHCO_3$. The resulting solid was collected by filtration under vacuum to afford 2-(pyridin-3-yl)-2-(4-(trifluoromethyl)pyridin-2-yl)acetamide as a white solid (0.6 g, 57%). LC-MS (ESI, m/z): 282.09 $[M+H]^+$.

Step 3: Preparation of 1-Amino-4-(pyridin-3-yl)-6-(trifluoromethyl)-3H-pyrido[1,2-c]pyrimidin-3-one

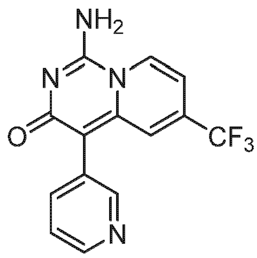

To a stirred solution of 2-(pyridin-3-yl)-2-(4-(trifluoromethyl)pyridin-2-yl)acetamide (0.3 g, 1.10 mmol, 1.00 equiv) in THF (10 mL) was added NaH (0.03 g, 1.6 mmol, 1.50 equiv, 60% in mineral oil) and the resulting mixture was stirred at RT for 10 min. Di(1*H*-imidazol-1-yl)methanimine (0.17 g, 1.10 mmol, 1.00 equiv) was added and the mixture was stirred at RT for 2 h. The reaction mixture concentrated and purified by prep-HPLC (mobile phase A - 0.1% aqueous formic acid; mobile phase B - MeCN; column - ATLANTIES T3 (19 x 250) mm, 5 mic; flow - 18mL/min; gradient method) to afford 1-amino-4-(pyridin-3-yl)-6-(trifluoromethyl)-3H-pyrido[1,2-c]pyrimidin-3-one (20 mg, 6%) as a yellow solid. $^1$HNMR (500 MHz, DMSO-$d_6$) $\delta$ 8.54 (dd, J = 5.0, 1.5 Hz, 1H), 8.47 (d, J = 1.5 Hz, 1H), 8.06 (d, J = 7.5 Hz, 1H), 7.80-7.70 (m, 3H), 7.46 (dd, J = 7.5, 5.0 Hz, 1H), 6.97 (s, 1H), 6.70 (dd, J = 7.5, 1.5 Hz, 1H). LC-MS (ESI, m/z): 307.1 $[M+H]^+$.

**Example 6:** Synthesis of 1-Amino-4-(1-methyl-1*H*-pyrazol-5-yl)-6-(trifluoromethyl)-3*H*-pyrido[1,2-c]pyrimidin-3-one

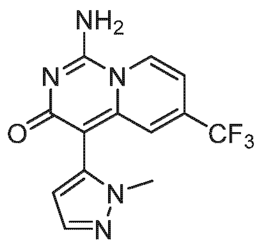

The title compound was prepared by following the procedure described in Example 5, by substituting 2-(pyridin-3-yl)acetonitrile with 2-(1-methyl-1H-pyrazol-5-yl)acetonitrile in step 1. $^1$HNMR (400 MHz, DMSO-$d_6$) δ 8.10 (d, J = 8.0 Hz, 1H), 7.81 (br s, 2H), 7.47 (d, J = 2.0 Hz, 1H), 7.06 (s, 1H), 6.76 (d, J = 8.0 Hz, 1H), 6.25 (d, J = 2.0 Hz, 1H). LC-MS (ESI, m/z): 310.1 [M+H]+.

**Example 7:** Synthesis of 1-Amino-6-bromo-4-phenyl-3*H*-pyrido[1,2-c]pyrimidin-3-one

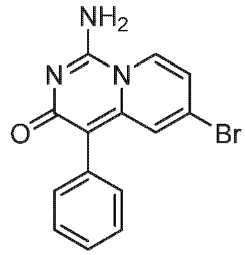

Step 1: Preparation of 2-(4-Bromopyridin-2-yl)-2-phenylacetonitrile

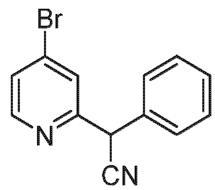

To a stirred solution of 2-phenylacetonitrile (1.00 g, 8.54 mmol, 1.00 equiv) in toluene (50 mL) at 0 °C was added KHMDS (8.54 mL, 8.54 mmol, 1.0 M in THF, 1.00 equiv) dropwise and the resulting mixture was stirred for 10 min. 4-Bromo-2-fluoropyridine (1.5 g, 8.54 mmol, 1.00 equiv) was added at the same temperature and stirring was continued at for 2 h at RT. The reaction mixture was cooled to 0 °C and water (20 mL) was added. The biphasic solution was acidified with 1 M aqueous HCl until pH ~5 and extracted with DCM (2 x 50 mL). The combined organic layers were dried over $Na_2SO_4$, filtered and concentrated. Purification by silica gel chromatography afforded 2-(4-bromopyridin-2-yl)-2-phenylacetonitrile (0.6 g, 25%) as a colorless oil. LC-MS (ESI, m/z): 273.1 $[M+H]^+$.

Step 2: Preparation of 1-Amino-6-bromo-4-phenyl-3*H*-pyrido[1,2-c]pyrimidin-3-one

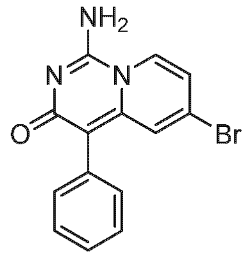

The title compound was prepared by following the procedure described in Example 5, by substituting 2-(pyridin-3-yl)-2-(4-(trifluoromethyl)pyridin-2-yl)acetonitrile with 2-(4-bromopyridin-2-yl)-2-phenylacetonitrile in step 2. 1HNMR (400 MHz, DMSO-d6) δ 7.85 (d, J = 5.6 Hz, 1H), 7.60-7.39 (m, 4H), 7.36-7.31 (m, 1H), 7.26-7.21 (m, 2H), 6.83 (d, J = 2.0 Hz, 1H), 6.61 (dd, J = 8.0, 2.0 Hz, 1H). LC-MS (ESI, m/z): 316.3 $[M+H]^+$.

**Example 8:** Synthesis of 1-Amino-6-bromo-4-(*o*-tolyl)-3*H*-pyrido[1,2-c]pyrimidin-3-one

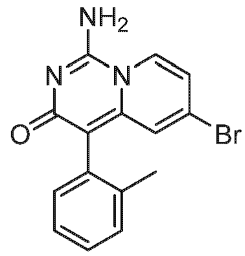

The title compound was prepared by following the procedure described in Example 7, by substituting 2-phenylacetonitrile with 2-(o-tolyl)acetonitrile in step 1. $^1$HNMR (400 MHz, DMSO-d6) δ 7.84 (d, J = 7.6 Hz, 1H), 7.53 (s, 2H), 7.32-7.20 (m, 3H), 7.16-7.13 (m, 1H), 6.61 (dd, J = 7.6, 2.0 Hz, 1H), 6.49 (d, J = 2.0 Hz, 1H), 2.04 (s, 3H). LC-MS (ESI, m/z): 330.2 $[M+H]^+$.

**Example 9:** Synthesis of*N*-(3-oxo-4-phenyl-6-(trifluoromethyl)-3*H*-pyrido[1,2-*c*]pyrimidin-1-yl)acetamide

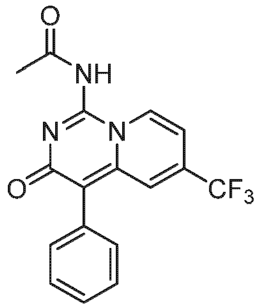

Step 1: Preparation of 1-Amino-4-phenyl-6-(trifluoromethyl)-3H-pyrido[1,2-c]pyrimidin-3-one

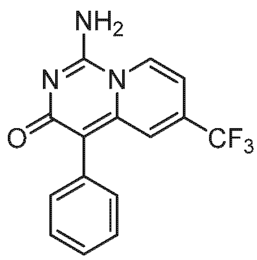

The title compound was prepared by following the same procedure described in Example 5, by substituting 2-(pyridin-3-yl)acetonitrile with 2-phenylacetonitrile in step 1. LC-MS (ESI, m/z): 306.11 $[M+H]^+$.

Step 2: Preparation of *N*-(3-oxo-4-phenyl-6-(trifluoromethyl)-3*H*-pyrido[1,2-*c*]pyrimidin-1-yl)acetamide

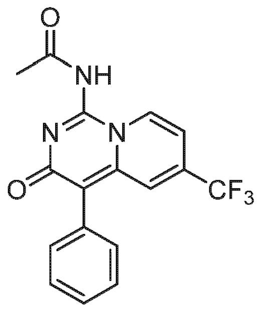

To a stirred solution of 1-amino-4-phenyl-6-(trifluoromethyl)-3H-pyrido[1,2-c]pyrimidin-3-one (200 mg, 0.65 mmol, 1.00 equiv) in DCM (5 mL) were added TEA (0.276 mL, 1.97 mmol, 3.00 equiv) and acetyl chloride (0.047 mL, 0.655 mmol, 1.00 equiv) at 0 °C and the resulting solution was stirred at RT for 16 h. The reaction mixture was diluted with water (20 mL) and extracted with DCM (2 x 30 mL). The combined organic layers were washed with brine (20 mL), dried over $Na_2SO_4$, filtered and concentrated. Purification by prep-HPLC (mobile phase A - 0.1% aqueous formic acid; mobile phase B - MeCN; column - X-BRIDGE C18 (19 x 250) mm, 5mic; flow - 18mL/min; gradient method) afforded N-(3-oxo-4-phenyl-6-(trifluoromethyl)-3*H*-pyrido[1,2-*c*]pyrimidin-1-yl)acetamide (20 mg, 9%) as an yellow solid. $^1$HNMR (400 MHz, DMSO-d6) δ 13.3 (br s, 1H), 8.85-8.80 (m, 1H), 7.50-7.45 (m, 3H), 7.38-7.33 (m, 2H), 7.08-7.04 (m, 1H), 6.79 (s, 1H), 2.25 (s, 3H). LC-MS (ESI, m/z): 348.4 $[M+H]^+$.

**Example 10:** Synthesis of 4-Phenyl-1-(propylamino)-6-(trifluoromethyl)-3H-pyrido[1,2-c]pyrimidin-3-one

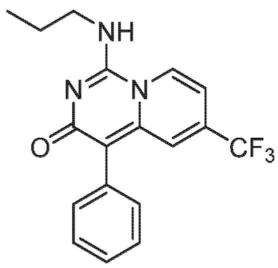

To a stirred solution of 1-amino-4-phenyl-6-(trifluoromethyl)-3*H*-pyrido[1,2-c]pyrimidin-3-one (100 mg, 0.33 mmol, 1.00 equiv) (product from step 1 in Example 1) in MeOH (5 mL) were added AcOH (2 drops) and propionaldehyde (95 mg, 1.64 mmol, 5.00 equiv) and the resulting solution was stirred for 1 hour at RT. 2-Picoline borane (70 mg, 0.655 mmol, 2.00 equiv) was added and the mixture was stirred for 16 h at RT. The reaction mixture was diluted with water (30 mL) and extracted with EtOAc (2 x 30 mL). The combined organic layers were washed with brine (20 mL), dried over $Na_2SO_4$, filtered and concentrated. Purification by prep-HPLC (mobile phase A - 0.1% aqueous formic acid; mobile phase B - MeCN; column - X-BRIDGE C18 (19 x 250) mm, 5mic; flow - 18mL/min; gradient method) afforded 4-phenyl-1-(propylamino)-6-(trifluoromethyl)-3*H*-pyrido[1,2-c]pyrimidin-3-one (30 mg, 26%) as an yellow solid. $^1$HNMR (400 MHz, DMSO-d6) δ 8.08 (d, J = 8.0 Hz, 1H), 7.91 (s, 1H), 7.46-7.41 (m, 2H), 7.38-7.35 (m, 1H), 7.30-7.25 (m, 2H), 6.96 (d, J = 2.0 Hz, 1H), 6.65 (dd, J = 8.0, 2.0 Hz, 1H), 3.38-3.30 (m, 2H), 1.70-1.58 (m, 2H), 0.94 (t, J = 7.2 Hz, 3H). LC-MS (ESI, m/z): 348.3 $[M+H]^+$.

**Example 11:** Synthesis of 1-(Methylamino)-4-phenyl-6-(trifluoromethyl)-3*H*-pyrido[1,2-c]pyrimidin-3-one

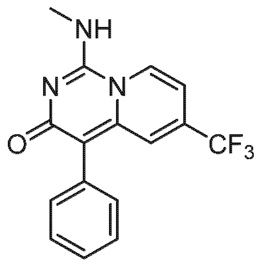

The title compound was prepared by following the procedure described in Example 10, by substituting propionaldehyde with formaldehyde. $^1$HNMR (400 MHz, DMSO-d6) δ 8.05 (d, J = 7.6 Hz, 1H), 8.02-7.90 (br s, 1H), 7.46-7.41 (m, 2H), 7.38-7.35 (m, 1H), 7.30-7.25 (m, 2H), 6.96 (d, J = 1.6 Hz, 1H), 6.63 (dd, J = 7.6, 1.6 Hz, 1H), 2.90 (s, 3H). LC-MS (ESI, m/z): 320.3 $[M+H]^+$.

**Biological Examples**

The ability of the compound of present disclosure to inhibit MAT2A enzyme was determined using a Phosphate Sensor Fluorescence Assay described below.

**Biologic Example A. Phosphate Sensor Fluorescence Assay**

MAT2A enzyme is incubated with a test compound in DMSO or DMSO and its substrates (L-methionine and ATP) in a microtiter plate. The enzymatic reaction is stopped by the addition of Working Phosphate Sensor Mixture. The plate is analyzed for fluorescence at 450 nm. The high control (DMSO with enzyme and its substrates) gives high fluorescence which represents no inhibition of enzymatic activity while the low control (DMSO with MAT2A substrates and no enzyme) gives low fluorescence which represents full inhibition of enzymatic activity.

**Materials:**

Human MAT2A: Cepter, amino acids 1-395

Tris, pH 7.5: Invitrogen cat # 15567-027

KCl: Ambion cat # AM9640G

$MgCl_2$: Ambion cat # AM9530G

Brij-35: Sigma cat B4184-10ML

DTT: Goldbio cat # DTT100

BGG: Sigma cat # G5009-25G

PNP: Novus Biologicals cat # NBP1-50872

7-MEG: Cayman Chemical cat # 15988

L-Methionine: Alfa Aesar cat # J61904

ATP: Alfa Aesar cat # J60336

Phosphate Sensor: Thermo Fisher cat # PV4407

EDTA: Life Tech cat # 15575-038

Assay plate: 384-well black polypropylene plate: Thomas Scientific cat # 1149Q35

**Final Assay Conditions:**

Assay Buffer: 50 mM Tris, pH 7.5/50 mM KCl/10 mM $MgCl_2$/0.01% Brij-35/1 mM DTT/0.1% BGG/40 nM PNP/6 uM 7-MEG

MAT2A: 10 nM for Cepter clone ID 329, lot 00023-123 before the addition of Working Phosphate Sensor Mixture
5 nM for Cepter clone ID 334, lot 00023-148 before the addition of Working Phosphate Sensor Mixture

L-methionine: 500 uM before the addition of Working Phosphate Sensor Mixture

ATP: 500 uM before the addition of Working Phosphate Sensor Mixture

**Procedure:**

For the assay, a mixture of 1 mM L-methionine/1 mM ATP (2X final pre-stopped concentration) in assay buffer; MAT2A (2X final pre-stopped concentration) in Assay Buffer and Working Phosphate Sensor Mixture (1.5 uM Phosphate Sensor/30 mM EDTA in Assay Buffer, which is 3X final concentrations) were prepared. Test compounds or DMSO were added to the appropriate well suing D300e digital dispenser. 5 μl/well of Assay Buffer was added to the wells corresponding to the negative control and 5 μl/well of MAT2A was added to all the wells except for those corresponding to the negative control. After incubating the plate at room temperature for 15 minutes, 5 μl/well of the 1 mM L-methionine/1 mM ATP mixture was added to all wells. The plate was centrifuged at 1000 rpm for 1 minute and then incubated at room temperature for 1 hour. 5 μl of the Working Phosphate Sensor Mixture was added to all wells and the plate was centrifuged at 1000 rpm for 1 minute. The plate was read for fluorescence at 450 nm after exciting at 430 nm.

**Data Analysis:**

Percent inhibition was calculated in Chemical and Biological Information System (CBIS), (ChemInnovation Software Inc.). Curves were fitted by CBIS as % inhibition vs. log [compound concentration] using a 4-parameter inhibition model.

$$\text{Fit} = (A+((B-A)/(1+((C/x)^D))))$$

$$\text{Res} = (y-\text{fit})$$

**Biologic Example B. Phosphate Sensor Fluorescence Assay**

MAT2A enzyme is incubated with a test compound in DMSO or DMSO and its substrates (L-methionine and ATP) in a microtiter plate. The enzymatic reaction is stopped by the addition of Working Phosphate Sensor Mixture. The plate is analyzed for fluorescence at 450 nm. The high control (DMSO with enzyme and its substrates) gives high fluorescence which represents no inhibition of enzymatic activity while the low control (DMSO with MAT2A substrates and no enzyme) gives low fluorescence which represents full inhibition of enzymatic activity.

**Materials:**

Human MAT2A: Cepter, amino acids 1-395 Clone ID 334, lot 00023-148

Tris, pH 7.5: Invitrogen cat # 15567-027

KCl: Ambion cat # AM9640G

$MgCl_2$: Ambion cat # AM9530G

Brij-35: Sigma cat B4184-10ML

DTT: Goldbio cat # DTT100

BGG: Sigma cat # G5009-25G

PNP: Novus Biologicals cat # NBP1-50872

7-MEG: Cayman Chemical cat # 15988

L-Methionine: JTBaker P/N 2085-05

ATP: Promega P/N V915B

Phosphate Sensor: Invitrogen cat # PV4407

EDTA: Life Tech cat # 15575-038

Assay plate: 384-well black polypropylene plate: Corning P/N 3573

**Final Assay Conditions:**

Assay Buffer: 50 mM Tris, pH 7.5/50 mM KCl/10 mM $MgCl_2$/0.01% Brij-35/1 mM DTT/0.01% BGG /40 nM PNP/6 uM 7-MEG

MAT2A: 5.8 nM MAT2A (1-395), Cepter, clone ID 334, lot 00023-148 before the addition of Working Phosphate Sensor Mixture

L-methionine: 500 uM before the addition of Working Phosphate Sensor Mixture

ATP: 500 uM before the addition of Working Phosphate Sensor Mixture

**Procedure:**

For the assay, a mixture of 1 mM L-methionine/1 mM ATP (2X final pre-stopped concentration) in assay buffer; MAT2A (2X final pre-stopped concentration) in Assay Buffer and Working Phosphate Sensor Mixture (1.5 uM Phosphate Sensor/30 mM EDTA in Assay Buffer, which is 3X final concentrations) were prepared. Test compounds or DMSO were added to the appropriate well using a Beckman Coulter Echo 550 acoustic liquid handler. 10 μL/well of Assay Buffer was added to the wells corresponding to the negative control and 10 μL/well of MAT2A was added to all the wells except for those corresponding to the negative control. After incubating the plate at room temperature for 15 minutes, 10 μL/well of the 1 mM L-methionine/1 mM ATP mixture was added to all wells. The plate was centrifuged at 1000 rpm for 1 minute and then incubated at room temperature for 1 hour. 10 μL of the Working Phosphate Sensor Mixture was added to all wells and the plate was centrifuged at 1000 rpm for 1 minute. The plate was read for fluorescence at 450 nm after exciting at 430 nm on a Tecan Spark microplate reader.

**Data Analysis:**

Percent inhibition was calculated in Chemical and Biological Information System (CBIS), (ChemInnovation Software Inc.). Curves were fitted by CBIS as % inhibition vs. log [compound concentration] using a 4-parameter inhibition model.

$$\text{Fit} = (A+((B-A)/(1+((C/x)^D))))$$

$$Res = (y\text{-}fit)$$

The $IC_{50}$ of a representative number of compounds in Table 1 above are disclosed in Table 3 below. Compounds 1.001-1.004 were tested using the procedure described in Bioogical Example A, described above. Compounds 1.005-1.011 were tested using the procedure described in Biological Example B, described above.
(+) $IC_{50}$= 10 μM-1 μM; 1 μM > (++) $IC_{50} \geq$ 500 nM; 500 nM > (+++) $IC_{50} \geq$ 200 nM; 200 nM > (++++) $IC_{50} \geq$ 10 nM; 10 nM > (+++++)

**Table 3**

| Cpd. No. (Table 1) | $IC_{50}$ |
|---|---|
| 1.001 | ++++ |
| 1.002 | ++++ |
| 1.003 | ++++ |
| 1.004 | +++ |
| 1.005 | ++++ |
| 1.006 | ++++ |
| 1.007 | ++++ |
| 1.008 | ++++ |
| 1.009 | + |
| 1.010 | ++++ |
| 1.011 | ++++ |

## Claims

1. A compound having the Formula (A)

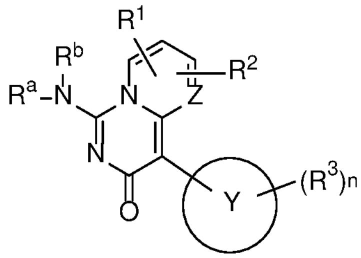

(A)

or a pharmaceutically acceptable salt thereof, wherein
Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, $C_{1-6}$ haloalkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, $C_{1-6}$ haloalkyl and -C(O)-$C_{1-4}$ alkyl; or

$R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl;
each $X^1$ is $C_{1-6}$ alkylene;

ring Y is a phenyl or a 5 to 6 membered heteroaryl comprising 1 to 3 heteroatom ring vertices independently selected from the group consisting of N, O, and S;
each $R^3$ is independently selected from the group consisting of halo, cyano, $C_{1-4}$ alkoxy, $C_{1-4}$ haloalkoxy, -OH, $C_{1-4}$ alkyl, and $C_{1-4}$ haloalkyl; and
the subscript n is 0, 1 or 2.

**2.** The compound of claim 1, having the Formula (I)

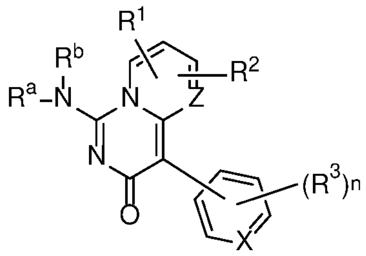

(I)

or a pharmaceutically acceptable salt thereof, wherein

Z is selected from the group consisting of CH and N;
$R^1$ and $R^2$ are each independently selected from the group consisting of H, $C_{1-6}$ alkyl, $C_{1-6}$ haloalkyl, $C_{1-6}$ alkoxy, cyano, halo, and $C_{3-8}$ cycloalkyl, wherein the cycloalkyl group is substituted with from 0 to 2 groups selected from the group consisting of $C_{1-4}$ alkyl and halo;
$R^a$ and $R^b$ are each independently selected from the group consisting of H, $C_{1-6}$ alkyl, and $C_{1-6}$ haloalkyl; or $R^a$ and $R^b$ together with the nitrogen to which they are attached combine to form a 4-to 6-membered heterocycloalkyl ring comprising 0 to 2 additional heteroatom ring vertices independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein each $R^x$ is selected from the group consisting of H, $C_{1-4}$ alkyl, and $C_{1-4}$ haloalkyl; each $X^1$ is $C_{1-6}$ alkylene;
X is CH, $CR^3$, or N;
each $R^3$ is independently selected from the group consisting of halo, $C_{1-4}$ alkyl, and $C_{1-4}$ haloalkyl; and
the subscript n is 0, 1 or 2.

**3.** The compound of claim 1 or claim 2, having Formula (Ia)

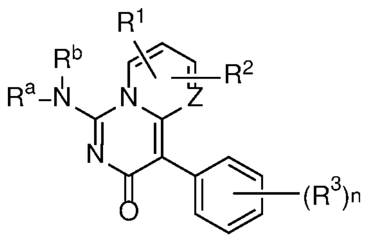

(Ia)

or a pharmaceutically acceptable salt thereof;

or having Formula (Ib)

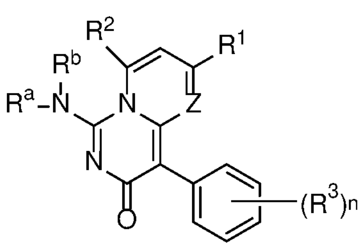

(Ib)

or a pharmaceutically acceptable salt thereof;
or having Formula (Ic)

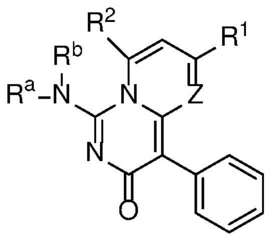
(Ic)

or a pharmaceutically acceptable salt thereof;
or having Formula (Id)

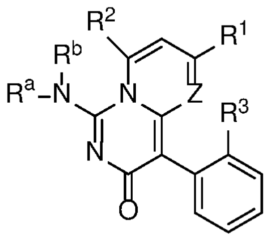
(Id)

or a pharmaceutically acceptable salt thereof;
or having Formula (Ie)

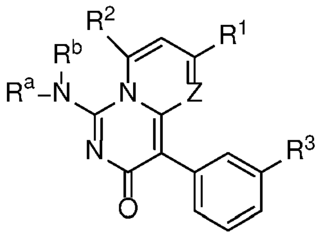
(Ie)

or a pharmaceutically acceptable salt thereof.

**4.** The compound of claim 1 or claim 2, having Formula (If)

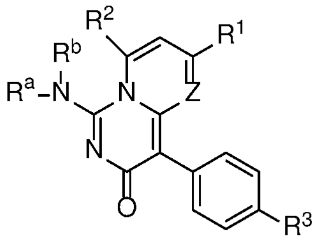
(If)

or a pharmaceutically acceptable salt thereof.

**5.** The compound of claim 1, wherein ring Y in Formula (A) is pyrazolyl; or wherein ring Y is pyridyl.

**6.** The compound of any one of claims 1 to 4, wherein Z is CH.

**7.** The compound of any one of claims 1 to 4, wherein Z is N.

**8.** The compound of any one of claims 1 to 7, wherein $R^1$ is selected from the group consisting of $C_{1-6}$ alkyl, $C_{1-6}$ haloalkyl, halo, and $C_{3-8}$ cycloalkyl, wherein the cycloalkyl group is substituted with from 0 to 2 groups selected from the group consisting of $C_{1-4}$ alkyl and halo;
or

wherein $R^1$ is selected from the group consisting of $C_{1-2}$ alkyl, $C_{1-2}$ haloalkyl, and halo;
or
wherein $R^1$ is selected from the group consisting of methyl, trifluoromethyl, chloro, bromo, fluoro, and cyclopropyl;
or
wherein $R^1$ is methyl;
or
wherein $R^1$ is trifluoromethyl;
or
wherein $R^1$ is chloro,
or
wherein $R^1$ is fluoro;
or
wherein $R^1$ is bromo;
or
wherein $R^1$ is cyclopropyl.

**9.** The compound of any one of claims 1 to 8, wherein $R^2$ is selected from the group consisting of H, $C_{1-2}$ alkyl, halo, and $C_{1-2}$ alkoxy;
or

wherein $R^2$ is selected from the group consisting of H and methoxy;
or
wherein $R^2$ is H;
or
wherein $R^2$ is methoxy.

**10.** The compound of any one of claims 1 to 9, wherein $R^a$ and $R^b$ are each independently selected from the group consisting of H, $C_{1-6}$ alkyl, and $C_{1-6}$ haloalkyl;
or

wherein $R^a$ and $R^b$ are each independently selected from the group consisting of H, $C_{1-2}$ alkyl, and $C_{1-2}$ haloalkyl;
or
wherein $R^a$ and $R^b$ are each H;
or
wherein $R^a$ and $R^b$ are each methyl;
or
wherein $R^a$ is H; and $R^b$ is methyl;
or
$R^a$ and $R^b$ together with the nitrogen to which they are attached combine to form a 4- to 6-membered heterocycloalkyl ring comprising 0 to 2 additional heteroatoms independently selected from the group consisting of N, O, and S, wherein the 4- to 6-membered heterocycloalkyl is substituted with 0 to 2 moieties independently selected from the group consisting of $C_{1-4}$ alkyl, $-OR^x$, and $-X^1-OR^x$, and wherein

each $R^x$ is selected from the group consisting of H, $C_{1-4}$ alkyl, and $C_{1-4}$ haloalkyl; and
each $X^1$ is $C_{1-6}$ alkylene;

or
wherein $R^a$ and $R^b$ together with the nitrogen to which they are attached combine to form a 4- to 6-membered heterocycloalkyl ring, wherein the 4- to 6-membered heterocycloalkyl is substituted with 0 to 2 moieties independently selected from the group consisting of $C_{1-4}$ alkyl, $-OR^x$, and $-X^1-OR^x$, wherein

each $R^x$ is selected from the group consisting of H, $C_{1-4}$ alkyl, and $C_{1-4}$ haloalkyl; and
each $X^1$ is $C_{1-6}$ alkylene;

or
wherein $R^a$ and $R^b$ together with the nitrogen to which they are attached combine to form a structure selected from the group consisting of

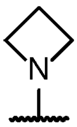, 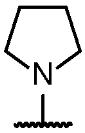, 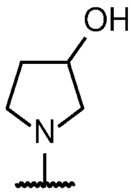, 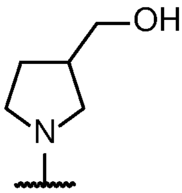, and 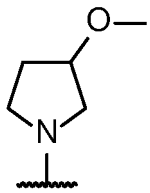.

**11.** The compound of any one of claims 1 to 10, wherein each $R^3$ is independently selected from the group consisting of cyano, $C_1$ haloalkoxy, $C_1$ haloalkyl, -OH, chloro, bromo, and methyl;
or

wherein each $R^3$ is independently selected from the group consisting of chloro, bromo, and methyl;
or
wherein each $R^3$ is chloro;
or
wherein each $R^3$ is bromo;
or
wherein each $R^3$ is methyl.

**12.** The compound of claim 1, wherein the compound is selected from a compound in Table X below

Table X

| Cpd. No. | **Structure** |
|---|---|
| **1.001** | 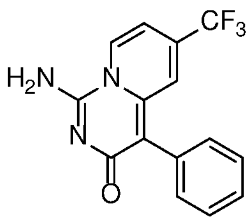 |
| **1.002** | 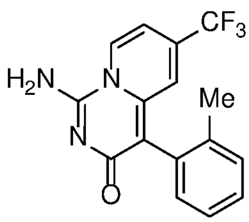 |
| **1.003** | 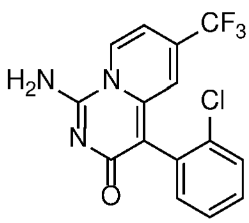 |

(continued)

| Cpd. No. | Structure |
|---|---|
| **1.004** | 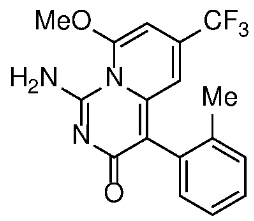 |
| **1.005** | 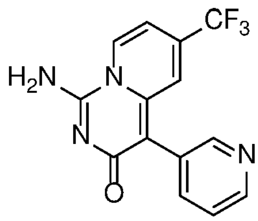 |
| **1.006** | 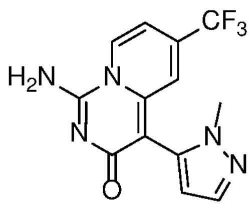 |
| **1.007** | 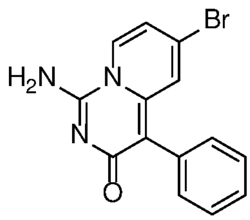 |
| **1.008** | 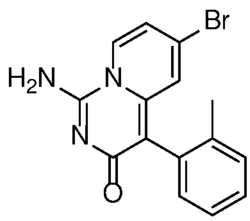 |
| **1.009** | 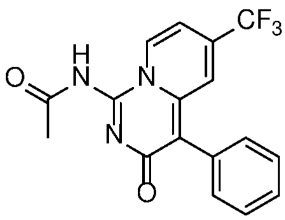 |
| **1.010** | 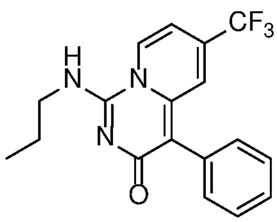 |

(continued)

| Cpd. No. | **Structure** |
|---|---|
| **1.011** | 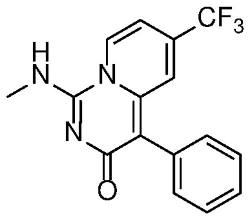 |

or a pharmaceutically acceptable salt thereof;
or Table Y below

Table Y

| Cpd. No. | **Structure** |
|---|---|
| **2.001** | 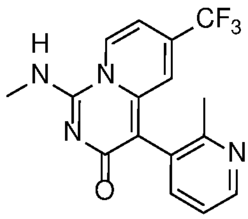 |
| **2.002** | 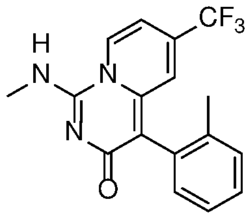 |
| **2.003** | 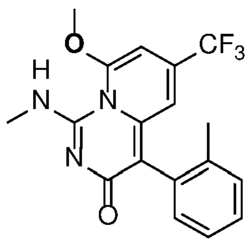 |
| **2.004** | 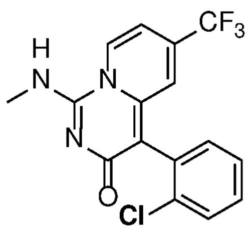 |
| **2.005** | 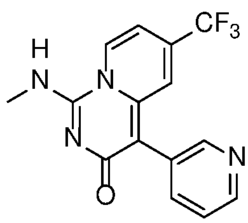 |

(continued)

| Cpd. No. | Structure |
|---|---|
| 2.006 | 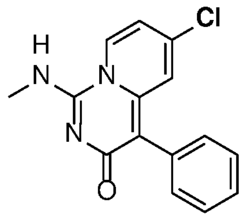 |
| 2.007 | 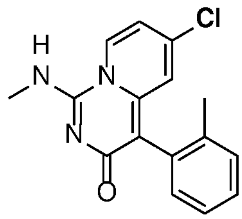 |
| 2.008 | 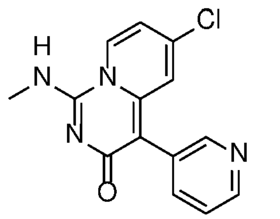 |
| 2.009 | 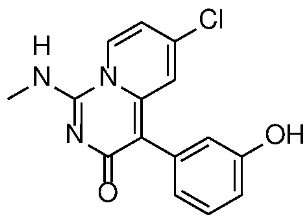 |
| 2.010 | 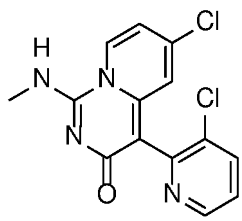 |
| 2.011 | 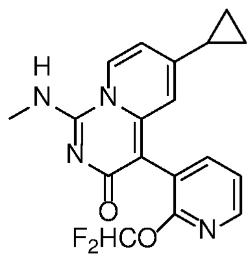 |
| 2.012 | 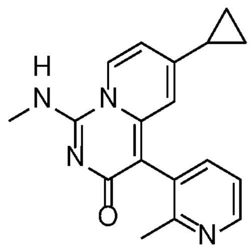 |

(continued)

| Cpd. No. | Structure |
|---|---|
| 2.013 | 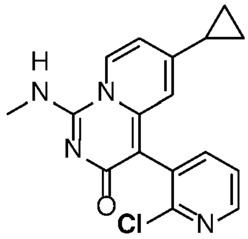 |
| 2.014 | 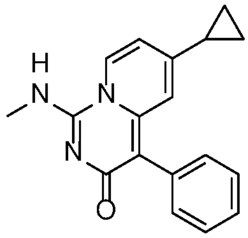 |
| 2.015 | 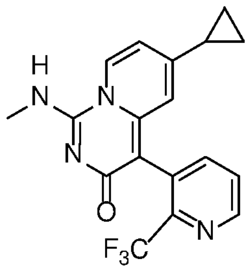 |
| 2.016 | 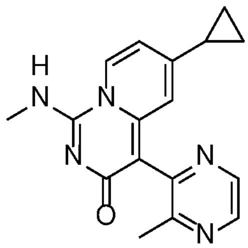 |
| 2.017 | 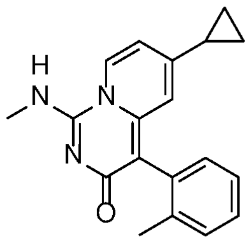 |
| 2.018 | 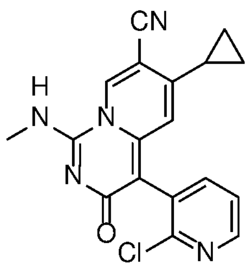 |

(continued)

| Cpd. No. | Structure |
|---|---|
| 2.019 | 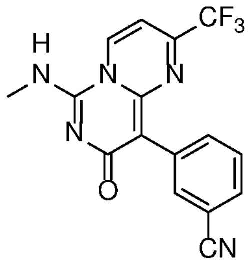 |
| 2.020 | 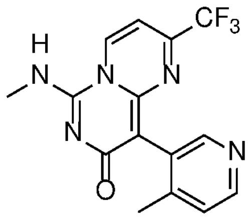 |
| 2.021 | 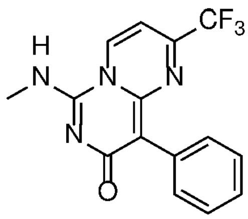 |
| 2.022 | 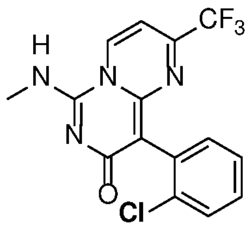 |
| 2.023 | 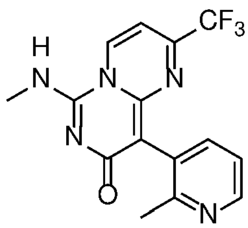 |
| 2.024 | 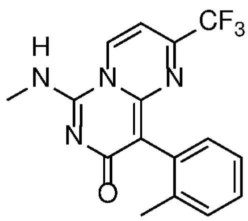 |
| 2.025 | 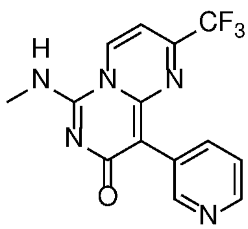 |

(continued)

| Cpd. No. | Structure |
|---|---|
| **2.026** | 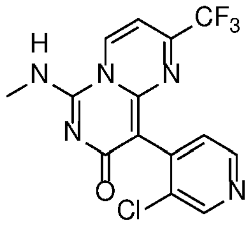 |
| **2.027** | 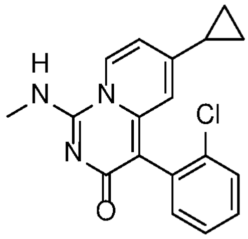 |

or a pharmaceutically acceptable salt thereof.

**13.** A pharmaceutical composition comprising a compound of any one of claims 1 to 12, or a pharmaceutically acceptable salt thereof and at least one pharmaceutically acceptable excipient.

**14.** A compound of any one of claims 1 to 12, for use in a method for treating a disease mediated by MAT2A in a patient comprising administering to the patient a therapeutically effective amount of: the compound, or the pharmaceutically acceptable salt thereof, wherein the disease is cancer,
preferably wherein the cancer is selected from the group consisting of leukemia, glioma, melanoma, pancreatic, non-small cell lung cancer, bladder cancer, astrocytoma, osteosarcoma, head and neck cancer, myxoid chondrosarcoma, ovarian cancer, endometrial cancer, breast cancer, soft tissue sarcoma, non-Hodgkin lymphoma and mesothelioma.

**15.** A compound of any one of claims 1 to 10, or a pharmaceutically acceptable salt thereof, for use in a method of treating a MTAP null cancer in a patient comprising administering to the patient a therapeutically effective amount of the compound; or the pharmaceutically acceptable salt thereof optionally in a pharmaceutical composition,
preferably wherein the cancer is selected from the group consisting of leukemia, glioma, melanoma, pancreatic, non-small cell lung cancer, bladder cancer, astrocytoma, osteosarcoma, head and neck cancer, myxoid chondrosarcoma, ovarian cancer, endometrial cancer, breast cancer, soft tissue sarcoma, non-Hodgkin lymphoma and mesothelioma.

**16.** A compound of any one of claims 1 to 10, or a pharmaceutically acceptable salt thereof, for use in a method for treating a cancer in a patient, wherein the cancer is **characterized by** a reduction or absence of MTAP gene expression, the absence of the MTAP gene, reduced level of MTAP protein, or reduced function of MTAP protein, comprising administering to the subject a therapeutically effective amount of the compound, or the pharmaceutically acceptable salt thereof optionally in a pharmaceutical composition,
preferably wherein the cancer is selected from the group consisting of leukemia, glioma, melanoma, pancreatic, non-small cell lung cancer, bladder cancer, astrocytoma, osteosarcoma, head and neck cancer, myxoid chondrosarcoma, ovarian cancer, endometrial cancer, breast cancer, soft tissue sarcoma, non-Hodgkin lymphoma and mesothelioma.

## Patentansprüche

**1.** Verbindung mit der Formel (A)

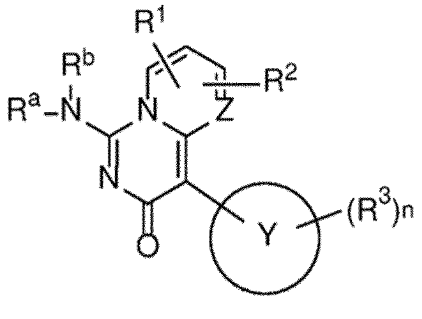

(A)

oder ein pharmazeutisch annehmbares Salz dieser, wobei Z ausgewählt ist aus der Gruppe bestehend aus CH und N;

$R^1$ und $R^2$ sind jeweils unabhängig voneinander ausgewählt aus der Gruppe bestehend aus H, $C_{1-6}$-Alkyl, $C_{1-6}$-Halogenalkyl, $C_{1-6}$-Alkoxy, $C_{1-6}$-Halogenalkoxy, Cyano, Halogen und $C_{3-8}$-Cycloalkyl, wobei die Cycloalkylgruppe mit 0 bis 2 Gruppen substituiert ist, die ausgewählt sind aus der Gruppe bestehend aus $C_{1-4}$-Alkyl und Halogen;

$R^a$ und $R^b$ sind jeweils unabhängig voneinander ausgewählt aus der Gruppe bestehend aus H, $C_{1-6}$-Alkyl, $C_{1-6}$-Halogenalkyl und -C(O)-$C_{1-4}$-Alkyl; oder $R^a$ und $R^b$ bilden zusammen mit dem Stickstoff, an den sie gebunden sind, einen 4- bis 6-gliedrigen Heterocycloalkylring, der 0 bis 2 zusätzliche Heteroatomring-Eckpunkte umfasst, die unabhängig voneinander ausgewählt sind aus der Gruppe bestehend aus N, O und S, wobei das 4- bis 6-gliedrige Heterocycloalkyl durch 0 bis 2 Reste substituiert ist, die unabhängig voneinander aus der Gruppe ausgewählt sind, die aus $C_{1-4}$-Alkyl, -$OR^x$ und -$X^1$-$OR^x$ besteht, und wobei jedes $R^x$ aus der Gruppe ausgewählt ist, die aus H, $C_{1-4}$-Alkyl und $C_{1-4}$-Halogenalkyl besteht; jedes $X^1$ ist $C_{1-6}$-Alkylen;

der Ring Y ist ein Phenyl oder ein 5- bis 6-gliedriges Heteroaryl, das 1 bis 3 Heteroatomring-Eckpunkte umfasst, die unabhängig voneinander aus der Gruppe ausgewählt sind, die aus N, O und S besteht;

jedes $R^3$ ist unabhängig voneinander aus der Gruppe ausgewählt, die aus Halogen, Cyano, $C_{1-4}$-Alkoxy, $C_{1-4}$-Halogenalkoxy, -OH, $C_{1-4}$-Alkyl und $C_{1-4}$-Halogenalkyl besteht; und

der Index n ist 0, 1 oder 2.

**2.** Verbindung nach Anspruch 1, mit der Formel (I)

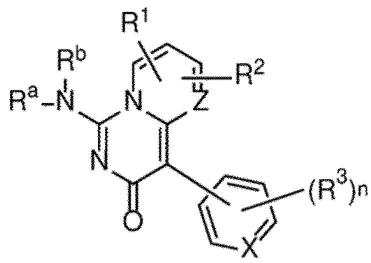

(I)

oder ein pharmazeutisch annehmbares Salz dieser, wobei Z ausgewählt ist aus der Gruppe bestehend aus CH und N;

$R^1$ und $R^2$ sind jeweils unabhängig voneinander ausgewählt aus der Gruppe bestehend aus H, $C_{1-6}$-Alkyl, $C_{1-6}$-Halogenalkyl, $C_{1-6}$-Alkoxy, Cyano, Halogen und $C_{3-8}$-Cycloalkyl, wobei die Cycloalkylgruppe mit 0 bis 2 Gruppen substituiert ist, die ausgewählt sind aus der Gruppe bestehend aus $C_{1-4}$-Alkyl und Halogen;

$R^a$ und $R^b$ sind jeweils unabhängig voneinander ausgewählt aus der Gruppe bestehend aus H, $C_{1-6}$-Alkyl, $C_{1-6}$-Halogenalkyl; oder

$R^a$ und $R^b$ bilden zusammen mit dem Stickstoff, an den sie gebunden sind, einen 4- bis 6-gliedrigen Heterocycloalkylring, der 0 bis 2 zusätzliche Heteroatomring-Eckpunkte umfasst, die unabhängig voneinander ausgewählt sind aus der Gruppe bestehend aus N, O und S, wobei das 4- bis 6-gliedrige Heterocycloalkyl durch 0 bis 2 Reste substituiert ist, die unabhängig voneinander aus der Gruppe ausgewählt sind, die aus $C_{1-4}$-Alkyl, -$OR^x$ und -$X^1$-$OR^x$ besteht, und wobei jedes $R^x$ aus der Gruppe ausgewählt ist, die aus H, $C_{1-4}$-Alkyl und $C_{1-4}$-Halogenalkyl besteht;

jedes $X^1$ ist $C_{1-6}$-Alkylen;

X ist CH, $CR^3$ oder N;
jedes $R^3$ ist unabhängig voneinander aus der Gruppe ausgewählt, die aus Halogen, $C_{1-4}$-Alkyl und $C_{1-4}$-Halogenalkyl besteht; und
der Index n ist 0, 1 oder 2.

**3.** Verbindung nach Anspruch 1 oder Anspruch 2, mit der Formel (Ia)

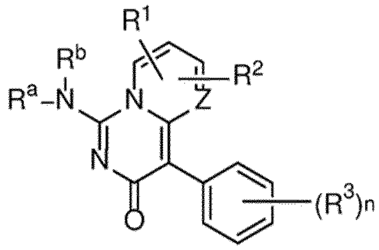

(Ia)

oder ein pharmazeutisch annehmbares Salz dieser;

oder mit der Formel (Ib)

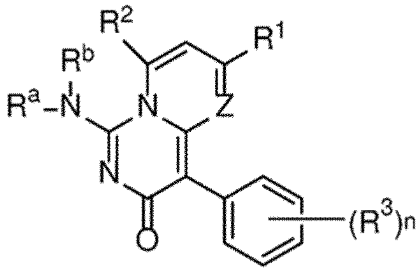

(Ib)

oder ein pharmazeutisch annehmbares Salz dieser;
oder mit der Formel (Ic)

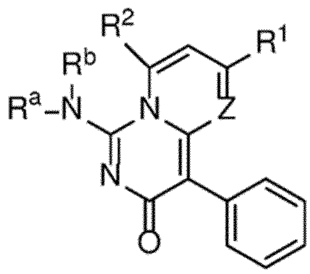

(Ic)

oder ein pharmazeutisch annehmbares Salz dieser;
oder mit der Formel (Id)

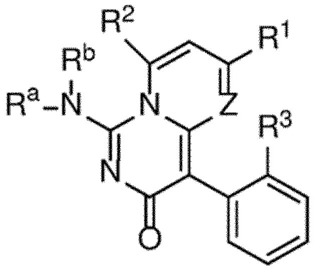

(Id)

oder ein pharmazeutisch annehmbares Salz dieser;
oder mit der Formel (Ie)

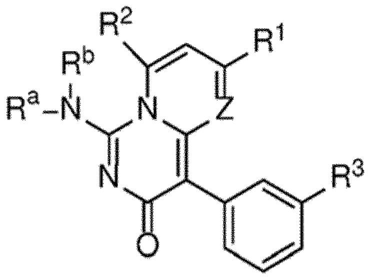

(Ie)

oder ein pharmazeutisch annehmbares Salz dieser.

**4.** Verbindung nach Anspruch 1 oder Anspruch 2, mit der Formel (If)

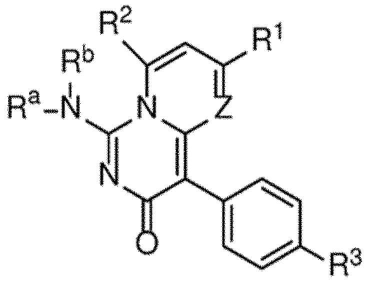

(If)

oder ein pharmazeutisch annehmbares Salz dieser.

**5.** Verbindung nach Anspruch 1, wobei der Ring Y in Formel (A) Pyrazolyl ist; oder
wobei der Ring Y Pyridyl ist.

**6.** Verbindung nach einem der Ansprüche 1 bis 4, wobei Z CH ist.

**7.** Verbindung nach einem der Ansprüche 1 bis 4, wobei Z N ist.

**8.** Verbindung nach einem der Ansprüche 1 bis 7, wobei $R^1$ ausgewählt ist aus der Gruppe bestehend aus $C_{1-6}$-Alkyl, $C_{1-6}$-Halogenalkyl, Halogen und $C_{3-8}$-Cycloalkyl, wobei die Cycloalkylgruppe mit 0 bis 2 Gruppen substituiert ist, die ausgewählt sind aus der Gruppe bestehend aus $C_{1-4}$-Alkyl und Halogen;
oder

wobei $R^1$ ausgewählt ist aus der Gruppe bestehend aus $C_{1-2}$-Alkyl, $C_{1-2}$-Halogenalkyl, und Halogen;
oder
wobei $R^1$ ausgewählt ist aus der Gruppe bestehend aus Methyl, Trifluormethyl, Chlor, Brom, Fluor und Cyclopropyl; oder
wobei $R^1$ Methyl ist;
oder
wobei $R^1$ Trifluormethyl ist;
oder
wobei $R^1$ Chlor ist,
oder
wobei $R^1$ Fluor ist;
oder
wobei $R^1$ Brom ist;
oder
wobei $R^1$ Cyclopropyl ist.

9. Verbindung nach einem der Ansprüche 1 bis 8, wobei $R^2$ ausgewählt ist aus der Gruppe bestehend aus H, $C_{1-2}$-Alkyl, Halogen und $C_{1-2}$-Alkoxy;
oder

wobei $R^2$ ausgewählt ist aus der Gruppe bestehend aus H und Methoxy;
oder
wobei $R^2$ H ist;
oder
wobei $R^2$ Methoxy ist.

10. Verbindung nach einem der Ansprüche 1 bis 9, wobei $R^a$ und $R^b$ jeweils unabhängig voneinander aus der Gruppe ausgewählt sind, die aus H, $C_{1-6}$-Alkyl und $C_{1-6}$-Halogenalkyl besteht;
oder

wobei $R^a$ und $R^b$ jeweils unabhängig voneinander aus der Gruppe ausgewählt sind, die aus H, $C_{1-2}$-Alkyl und $C_{1-2}$-Halogenalkyl besteht;
oder
wobei $R^a$ und $R^b$ jeweils H sind;
oder
wobei $R^a$ und $R^b$ jeweils Methyl sind;
oder
wobei $R^a$ H ist; und $R^b$ Methyl ist;
oder
$R^a$ und $R^b$ zusammen mit dem Stickstoff, an den sie gebunden sind, einen 4- bis 6-gliedrigen Heterocycloalkylring bilden, der 0 bis 2 zusätzliche Heteroatome umfasst, die unabhängig voneinander aus der Gruppe ausgewählt sind, die aus N, O und S besteht, wobei das 4- bis 6-gliedrige Heterocycloalkyl mit 0 bis 2 Resten substituiert ist, die unabhängig voneinander ausgewählt sind aus der Gruppe bestehend aus $C_{1-4}$-Alkyl, $-OR^x$ und $-X^1-OR^x$, und wobei
jedes $R^x$ ausgewählt ist aus der Gruppe bestehend aus H, $C_{1-4}$-Alkyl und $C_{1-4}$-Halogenalkyl; und
jedes $X^1$ ist $C_{1-6}$-Alkylen;
oder
wobei $R^a$ und $R^b$ zusammen mit dem Stickstoff, an den sie gebunden sind, einen 4- bis 6-gliedrigen Heterocycloalkylring bilden, wobei das 4- bis 6-gliedrige Heterocycloalkyl mit 0 bis 2 Resten substituiert ist, die unabhängig voneinander ausgewählt sind aus der Gruppe bestehend aus $C_{1-4}$-Alkyl, $-OR^x$ und $-X^1-OR^x$, wobei jedes $R^x$ ausgewählt ist aus der Gruppe bestehend aus H, $C_{1-4}$-Alkyl und $C_{1-4}$-Halogenalkyl; und
jedes $X^1$ $C_{1-6}$-Alkylen ist;
oder
wobei $R^a$ und $R^b$ zusammen mit dem Stickstoff, an den sie gebunden sind, eine Struktur bilden, ausgewählt aus der Gruppe bestehend aus

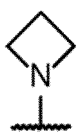 , 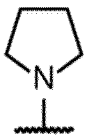 , 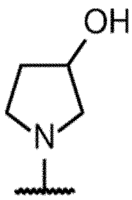 , 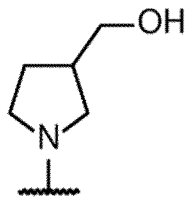 , und 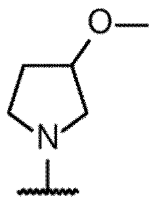 .

11. Verbindung nach einem der Ansprüche 1 bis 10, wobei jedes $R^3$ unabhängig voneinander ausgewählt ist aus der Gruppe bestehend aus Cyano, $C_1$-Halogenalkoxy, $C_1$-Halogenalkyl, -OH, Chlor, Brom und Methyl;
oder

wobei jedes $R^3$ unabhängig voneinander aus der Gruppe ausgewählt ist, die aus Chlor, Brom und Methyl besteht;
oder
wobei jedes $R^3$ Chlor ist;
oder
wobei jedes $R^3$ Brom ist;
oder

wobei jedes $R^3$ Methyl ist.

**12.** Verbindung nach Anspruch 1, wobei die Verbindung ausgewählt ist aus einer Verbindung in der nachstehenden Tabelle X

Tabelle X

| Verbdg. Nr. | **Struktur** |
|---|---|
| **1.001** | 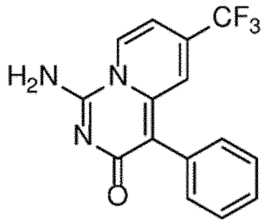 |
| **1.002** | 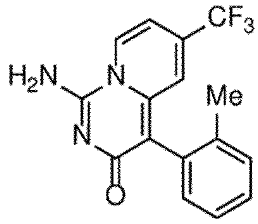 |
| **1.003** | 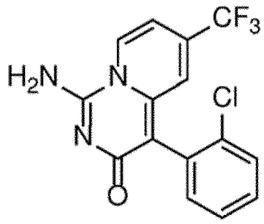 |
| **1.004** | 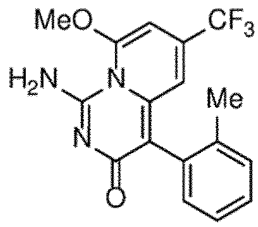 |
| **1.005** | 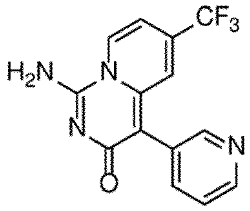 |
| **1.006** | 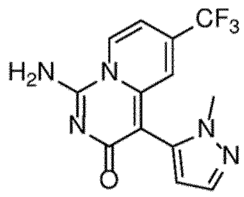 |

(continued)

| Verbdg. Nr. | **Struktur** |
|---|---|
| **1.007** | 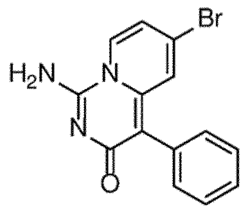 |
| **1.008** | 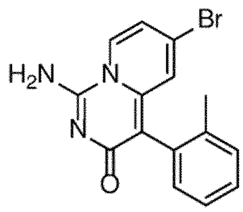 |
| **1.009** | 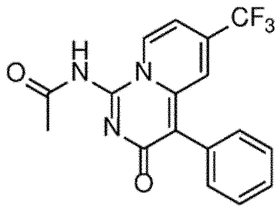 |
| **1.010** | 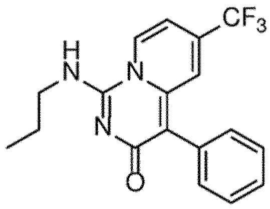 |
| **1.011** | 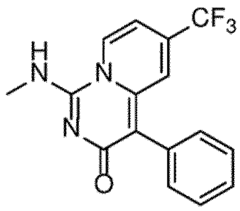 |

oder ein pharmazeutisch annehmbares Salz dieser;
oder der nachstehenden Tabelle Y

Tabelle Y

| Verbdg. Nr. | **Struktur** |
|---|---|
| **2.001** | 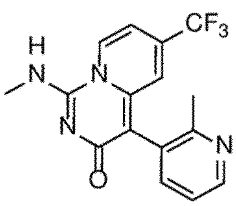 |

(continued)

| Verbdg. Nr. | **Struktur** |
|---|---|
| **2.002** | 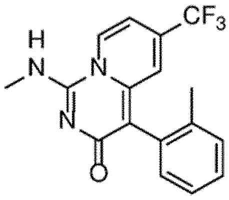 |
| **2.003** | 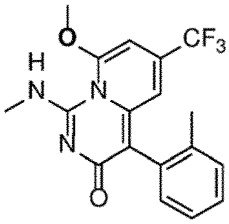 |
| **2.004** | 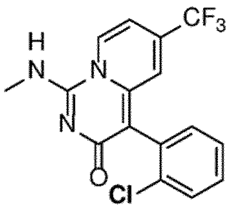 |
| **2.005** | 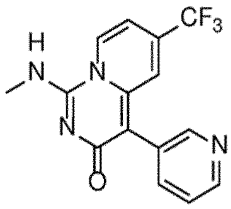 |
| **2.006** | 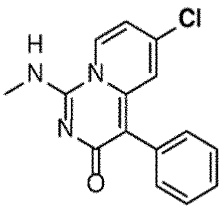 |
| **2.007** | 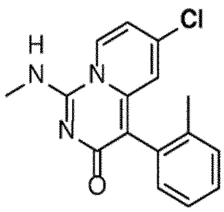 |
| **2.008** | 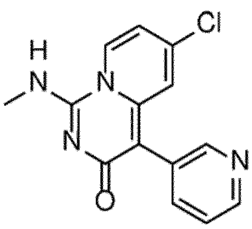 |

(continued)

| Verbdg. Nr. | **Struktur** |
|---|---|
| **2.009** | 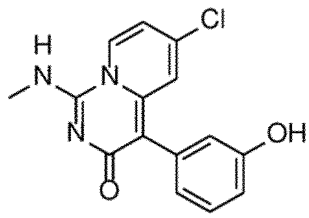 |
| **2.010** | 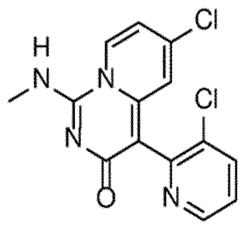 |
| **2.011** | 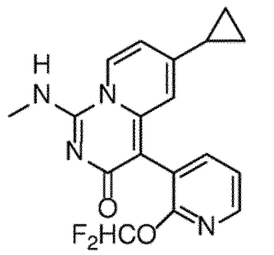 |
| **2.012** | 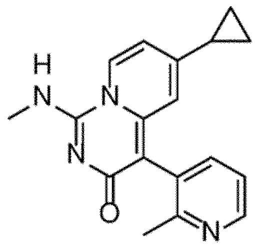 |
| **2.013** | 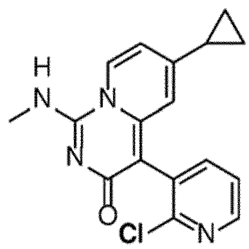 |
| **2.014** | 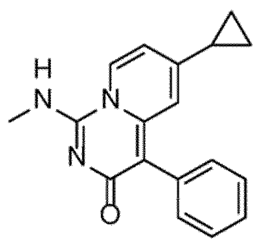 |

(continued)

| Verbdg. Nr. | **Struktur** |
|---|---|
| **2.015** | 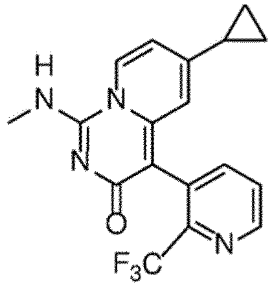 |
| **2.016** | 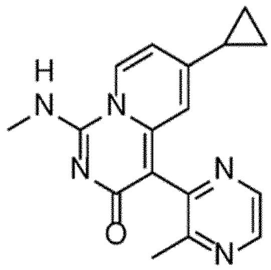 |
| **2.017** | 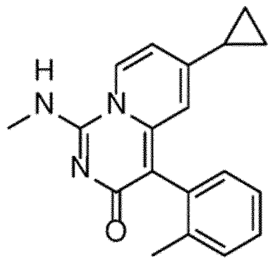 |
| **2.018** | 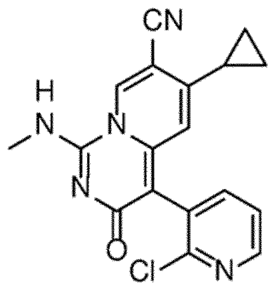 |
| **2.019** | 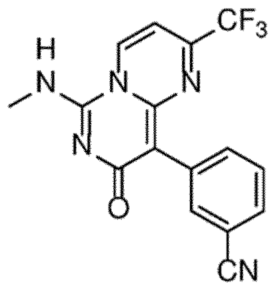 |

(continued)

| Verbdg. Nr. | **Struktur** |
|---|---|
| **2.020** | 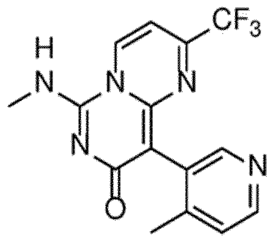 |
| **2.021** | 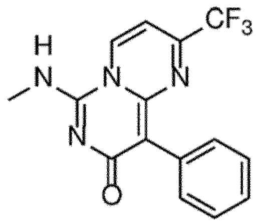 |
| **2.022** | 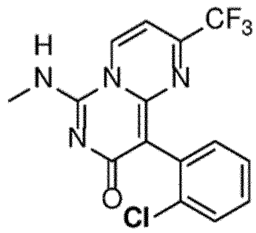 |
| **2.023** | 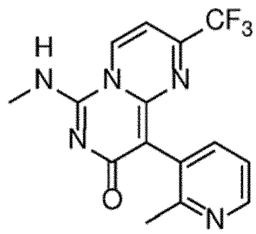 |
| **2.024** | 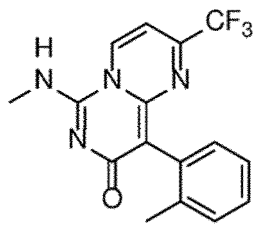 |
| **2.025** | 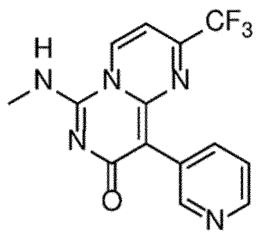 |

(continued)

| Verbdg. Nr. | **Struktur** |
|---|---|
| **2.026** | 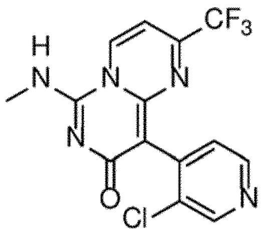 |
| **2.027** | 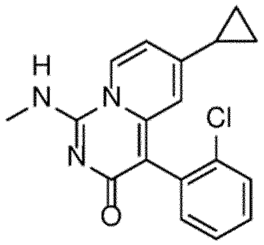 |

oder ein pharmazeutisch annehmbares Salz dieser.

**13.** Pharmazeutische Zusammensetzung, umfassend eine Verbindung nach einem der Ansprüche 1 bis 12 oder ein pharmazeutisch annehmbares Salz dieser und mindestens einen pharmazeutisch annehmbaren Hilfsstoff.

**14.** Verbindung nach einem der Ansprüche 1 bis 12 zur Anwendung in einem Verfahren zur Behandlung einer durch MAT2A vermittelten Erkrankung bei einem Patienten, umfassend die Verabreichung einer therapeutisch wirksamen Menge der Verbindung oder des pharmazeutisch annehmbaren Salzes dieser an den Patienten, wobei die Erkrankung Krebs ist,
vorzugsweise wobei der Krebs ausgewählt ist aus der Gruppe bestehend aus Leukämie, Gliom, Melanom, Pankreaskrebs, nicht-kleinzelligem Lungenkrebs, Blasenkrebs, Astrozytom, Osteosarkom, Kopf-Hals-Karzinom, myxoidem Chondrosarkom, Eierstockkrebs, Endometriumkarzinom, Brustkrebs, Weichteilsarkom, Non-Hodgkin-Lymphom und Mesotheliom.

**15.** Verbindung nach einem der Ansprüche 1 bis 10 oder ein pharmazeutisch annehmbares Salz dieser zur Anwendung in einem Verfahren zur Behandlung eines MTAP-negativen Krebses bei einem Patienten, umfassend das Verabreichen einer therapeutisch wirksamen Menge der Verbindung an den Patienten; oder des pharmazeutisch annehmbaren Salzes dieser, optional in einer pharmazeutischen Zusammensetzung,
vorzugsweise wobei der Krebs ausgewählt ist aus der Gruppe bestehend aus Leukämie, Gliom, Melanom, Pankreaskrebs, nicht-kleinzelligem Lungenkrebs, Blasenkrebs, Astrozytom, Osteosarkom, Kopf-Hals-Karzinom, myxoidem Chondrosarkom, Eierstockkrebs, Endometriumkarzinom, Brustkrebs, Weichteilsarkom, Non-Hodgkin-Lymphom und Mesotheliom.

**16.** Verbindung nach einem der Ansprüche 1 bis 10 oder ein pharmazeutisch annehmbares Salz dieser zur Anwendung in einem Verfahren zur Behandlung eines Krebses bei einem Patienten, wobei der Krebs durch eine Verringerung oder das Fehlen der MTAP-Genexpression, das Fehlen des MTAP-Gens, einen verringerten MTAP-Proteingehalt oder eine verringerte Funktion des MTAP-Proteins gekennzeichnet ist, umfassend das Verabreichen einer therapeutisch wirksamen Menge der Verbindung oder des pharmazeutisch annehmbaren Salzes dieser, optional in einer pharmazeutischen Zusammensetzung, an das Subjekt,
vorzugsweise wobei der Krebs ausgewählt ist aus der Gruppe bestehend aus Leukämie, Gliom, Melanom, Pankreaskrebs, nicht-kleinzelligem Lungenkrebs, Blasenkrebs, Astrozytom, Osteosarkom, Kopf-Hals-Karzinom, myxoidem Chondrosarkom, Eierstockkrebs, Endometriumkarzinom, Brustkrebs, Weichteilsarkom, Non-Hodgkin-Lymphom und Mesotheliom.

**Revendications**

1. Composé de formule (A)

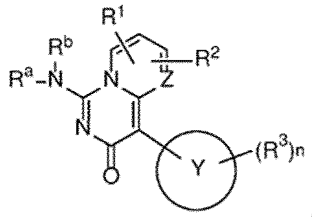

(A)

ou un sel pharmaceutiquement acceptable de celui-ci, dans lequel

Z est sélectionné dans le groupe consistant en CH et N ;
$R^1$ et $R^2$ sont chacun indépendamment choisis dans le groupe consistant en H, un alkyle en $C_{1-6}$, un haloalkyle en $C_{1-6}$, un alcoxy en $C_{1-6}$, un haloalcoxy en $C_{1-6}$, un cyano, un halo, et un cycloalkyle en $C_{3-8}$, dans lequel le groupe cycloalkyle est substitué avec 0 à 2 groupes choisis dans le groupe consistant en un alkyle en $C_{1-4}$ et un halo ;
$R^a$ et $R^b$ sont chacun indépendamment choisis dans le groupe consistant en H, un alkyle en $C_{1-6}$, un haloalkyle en $C_{1-6}$ et un -C(O)-alkyle en $C_{1-4}$ ; ou
$R^a$ et $R^b$ conjointement avec l'azote auquel ils sont liés se combinent pour former un cycle hétérocycloalkyle de 4 à 6 chaînons comprenant 0 à 2 hétéroatomes supplémentaires en sommet de cycle choisis indépendamment dans le groupe consistant en N, O, et S, dans lequel l'hétérocycloalkyle de 4 à 6 chaînons est substitué avec 0 à 2 fragments choisis indépendamment dans le groupe consistant en un alkyle en $C_{1-4}$, $-OR^X$, et $-X^1-OR^X$, et dans lequel chaque $R^X$ est choisi dans le groupe consistant en H, un alkyle en $C_{1-4}$ et un haloalkyle en $C_{1-4}$ ;
chaque $X^1$ est un alkylène en $C_{1-6}$ ;
le cycle Y est un phényle ou un hétéroaryle à 5 à 6 chaînons comprenant 1 à 3 hétéroatomes en sommet de cycle choisis indépendamment dans le groupe consistant en N, O et S ;
chaque $R^3$ est choisi indépendamment dans le groupe consistant en un halo, un cyano, un alcoxy en $C_{1-4}$, un haloalcoxy en $C_{1-4}$, -OH, un alkyle en $C_{1-4}$ et un haloalkyle en $C_{1-4}$ ; et
l'indice n est 0, 1 ou 2.

2. Composé selon la revendication 1, de formule (I)

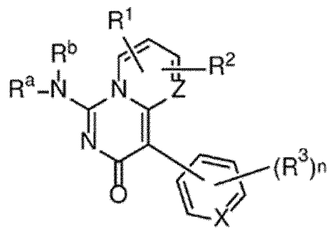

(I)

ou un sel pharmaceutiquement acceptable de celui-ci, dans lequel

Z est sélectionné dans le groupe consistant en CH et N ;
$R^1$ et $R^2$ sont chacun indépendamment choisis dans le groupe consistant en H, un alkyle en $C_{1-6}$, un haloalkyle en $C_{1-6}$, un alcoxy en $C_{1-6}$, un cyano, un halo, et un cycloalkyle en $C_{3-8}$, dans lequel le groupe cycloalkyle est substitué avec 0 à 2 groupes choisis dans le groupe consistant en un alkyle en $C_{1-4}$ et un halo ;
$R^a$ et $R^b$ sont chacun indépendamment choisis dans le groupe consistant en H, un alkyle en $C_{1-6}$, et un haloalkyle en $C_{1-6}$ ; ou
$R^a$ et $R^b$ conjointement avec l'azote auquel ils sont liés se combinent pour former un cycle hétérocycloalkyle de 4 à 6 chaînons comprenant 0 à 2 hétéroatomes supplémentaires en sommet de cycle choisis indépendamment dans le groupe consistant en N, O, et S, dans lequel l'hétérocycloalkyle de 4 à 6 chaînons est substitué avec 0 à 2 fragments choisis indépendamment dans le groupe consistant en un alkyle en $C_{1-4}$, $-OR^X$, et $-X^1-OR^X$, et dans

lequel chaque $R^X$ est choisi dans le groupe consistant en H, un alkyle en $C_{1-4}$ et un haloalkyle en $C_{1-4}$ ;
chaque $X^1$ est un alkylène en $C_{1-6}$ ;
X est CH, $CR^3$, ou N ;
chaque $R^3$ est choisi indépendamment dans le groupe consistant en un halo, un alkyle en $C_{1-4}$ et un haloalkyle en $C_{1-4}$ ; et
l'indice n est 0, 1 ou 2.

**3.** Composé selon la revendication 1 ou la revendication 2, de formule (Ia)

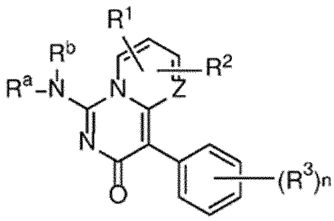

(Ia)

ou un sel pharmaceutiquement acceptable de celui-ci ;

ou de formule (Ib)

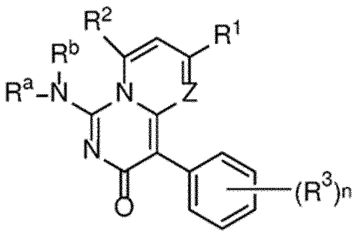

(Ib)

ou un sel pharmaceutiquement acceptable de celui-ci ;
ou de formule (Ic)

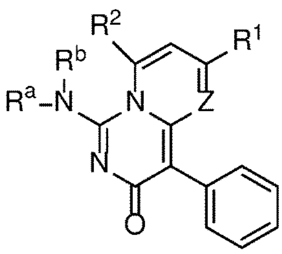

(Ic)

ou un sel pharmaceutiquement acceptable de celui-ci ;
ou de formule (Id)

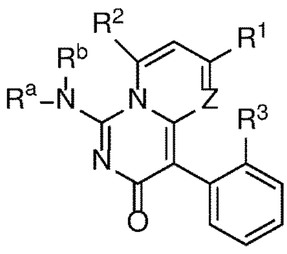

(Id)

ou un sel pharmaceutiquement acceptable de celui-ci ;

ou de formule (Ie)

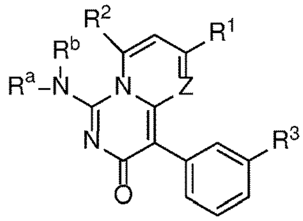

(Ie)

ou un sel pharmaceutiquement acceptable de celui-ci.

4. Composé selon la revendication 1 ou la revendication 2, de formule (If)

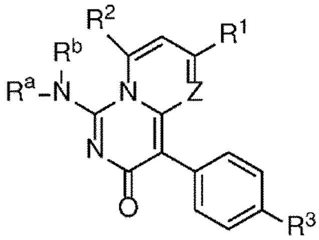

(If)

ou un sel pharmaceutiquement acceptable de celui-ci.

5. Composé selon la revendication 1, dans lequel le cycle Y de formule (A) est un pyrazolyle ; ou dans laquelle le cycle Y est un pyridyle.

6. Composé selon l'une quelconque des revendications 1 à 4, dans lequel Z est CH.

7. Composé selon l'une quelconque des revendications 1 à 4, dans lequel Z est N.

8. Composé selon l'une quelconque des revendications 1 à 7, dans lequel $R^1$ est choisi dans le groupe consistant en un alkyle en $C_{1-6}$, un haloalkyle en $C_{1-6}$, un halo et un cycloalkyle en $C_{3-8}$, dans lequel le groupe cycloalkyle est substitué avec 0 à 2 groupes choisis dans le groupe consistant en un alkyle en $C_{1-4}$ et un halo ;
ou

dans lequel $R^1$ est choisi dans le groupe consistant en un alkyle en $C_{1-2}$, un haloalkyle en $C_{1-2}$ et un halo ;
ou
dans lequel $R^1$ est choisi dans le groupe consistant en un méthyle, un trifluorométhyle, un chloro, un bromo, un fluoro et un cyclopropyle ; ou
dans lequel $R^1$ est un méthyle ;
ou
dans lequel $R^1$ est un trifluorométhyle ;
ou
dans lequel $R^1$ est un chloro,
ou
dans lequel $R^1$ est un fluoro ;
ou
dans lequel $R^1$ est un bromo ;
ou
dans lequel $R^1$ est un cyclopropyle.

9. Composé selon l'une quelconque des revendications 1 à 8, dans lequel $R^2$ est choisi dans le groupe consistant en H, un alkyle en $C_{1-2}$, un halo et un alcoxy en $C_{1-2}$ ;
ou

dans lequel $R^2$ est choisi dans le groupe consistant en H et un méthoxy ;
ou
dans lequel $R^2$ est H ;
ou
dans lequel $R^2$ est un méthoxy.

**10.** Composé selon l'une quelconque des revendications 1 à 9, dans lequel $R^a$ et $R^b$ sont chacun indépendamment choisis dans le groupe consistant en H, un alkyle en $C_{1-6}$ et un haloalkyle en $C_{1-6}$ ;
ou

dans lequel $R^a$ et $R^b$ sont chacun indépendamment choisis dans le groupe consistant en H, un alkyle en $C_{1-2}$, et un haloalkyle en $C_{1-2}$ ;
ou
dans lequel $R^a$ et $R^b$ sont chacun H ;
ou
dans lequel $R^a$ et $R^b$ sont chacun un méthyle ;
ou
dans lequel $R^a$ est H ; et $R^b$ est un méthyle ;
ou
$R^a$ et $R^b$ conjointement avec l'azote auquel ils sont liés se combinent pour former un cycle hétérocycloalkyle de 4 à 6 chaînons comprenant 0 à 2 hétéroatomes supplémentaires choisis indépendamment dans le groupe consistant en N, O, et S, dans lequel l'hétérocycloalkyle de 4 à 6 chaînons est substitué avec 0 à 2 fragments choisis indépendamment dans le groupe consistant en un alkyle en $C_{1-4}$, $-OR^x$, et $-X^1-OR^x$, et dans lequel chaque $R^x$ est choisi dans le groupe consistant en H, un alkyle en $C_{1-4}$ et un haloalkyle en $C_{1-4}$ ; et chaque $X^1$ est un alkylène en $C_{1-6}$ ;
ou
dans lequel $R^a$ et $R^b$ conjointement avec l'azote auquel ils sont liés se combinent pour former un cycle hétérocycloalkyle de 4 à 6 chaînons, dans lequel l'hétérocycloalkyle de 4 à 6 chaînons est substitué avec 0 à 2 fragments choisis indépendamment dans le groupe consistant en un alkyle en $C_{1-4}$, $-OR^x$, et $-X^1-OR^x$, dans lequel
chaque $R^X$ est choisi dans le groupe consistant en H, un alkyle en $C_{1-4}$ et un haloalkyle en $C_{1-4}$ ; et chaque $X^1$ est un alkylène en $C_{1-6}$ ;
ou
dans lequel $R^a$ et $R^b$ conjointement avec l'azote auquel ils sont liés se combinent pour former une structure choisie dans le groupe consistant en

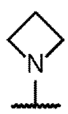 , 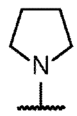 , 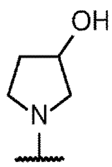 , 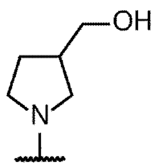 , et 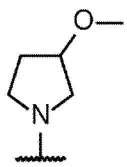

**11.** Composé selon l'une quelconque des revendications 1 à 10, dans lequel chaque $R^3$ est choisi indépendamment dans le groupe consistant en un cyano, un haloalcoxy en $C_1$, un haloalkyle en $C_1$, -OH, un chloro, un bromo et un méthyle ;
ou

dans lequel chaque $R^3$ est choisi indépendamment dans le groupe consistant en un chloro, un bromo et un méthyle ;
ou
dans lequel chaque $R^3$ est un chloro ;
ou
dans lequel chaque $R^3$ est un bromo ;
ou
dans lequel chaque $R^3$ est un méthyle.

**12.** Composé selon la revendication 1, dans lequel le composé est choisi parmi un composé du tableau X ci-dessous

Tableau X

| Composé numéro | **Structure** |
|---|---|
| **1.001** | 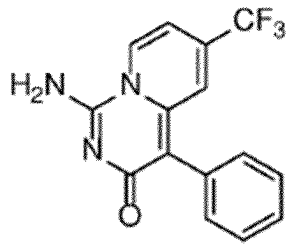 |
| **1.002** | 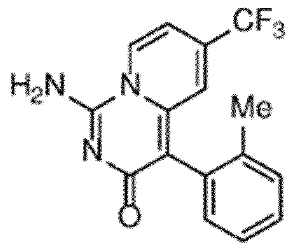 |
| **1.003** | 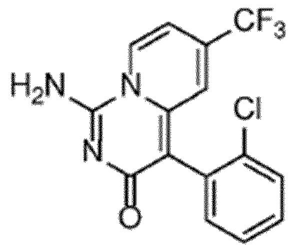 |
| **1.004** | 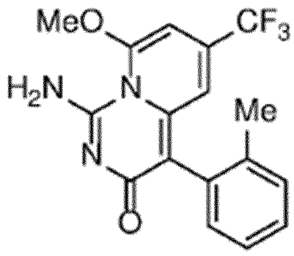 |
| **1.005** | 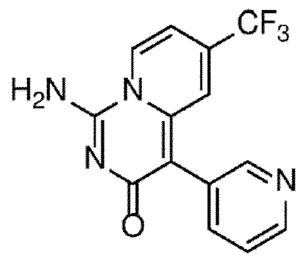 |
| **1.006** | 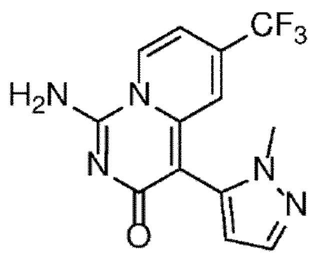 |

(continued)

| Composé numéro | **Structure** |
|---|---|
| **1.007** | 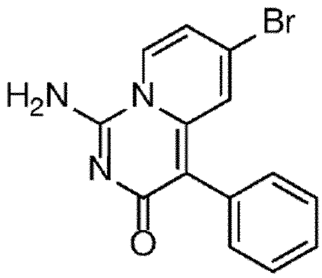 |
| **1.008** | 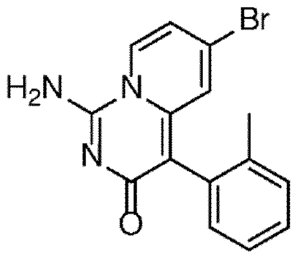 |
| **1.009** | 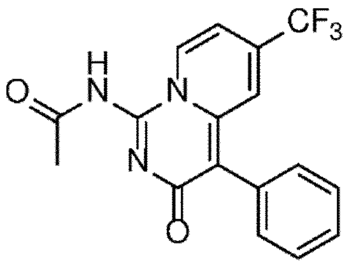 |
| **1.010** | 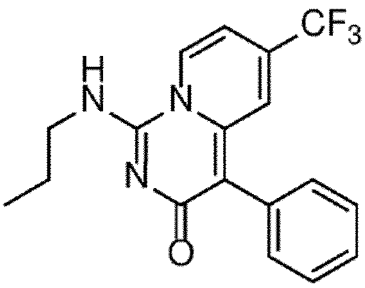 |
| **1.011** | 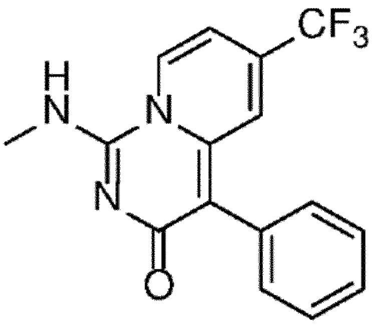 |

ou un sel pharmaceutiquement acceptable de ceux-ci ;
ou du tableau Y ci-dessous

Tableau Y

| Composé numéro | **Structure** |
|---|---|
| **2.001** | 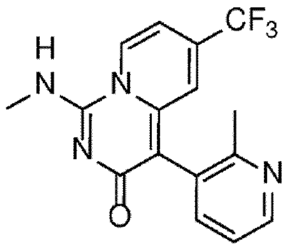 |
| **2.002** | 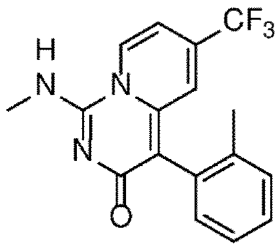 |
| **2.003** | 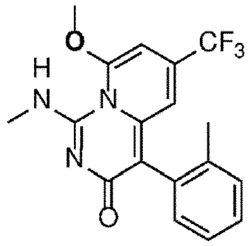 |
| **2.004** | 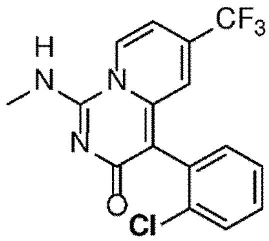 |
| **2.005** | 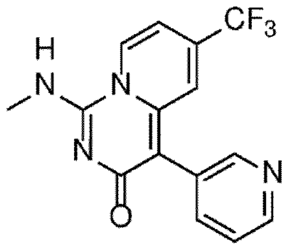 |
| **2.006** | 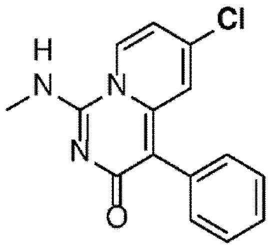 |

(continued)

| Composé numéro | **Structure** |
|---|---|
| **2.007** | 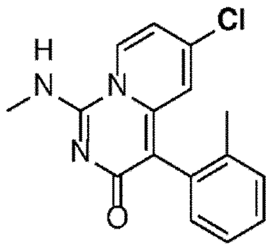 |
| **2.008** | 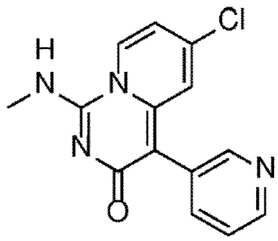 |
| **2.009** | 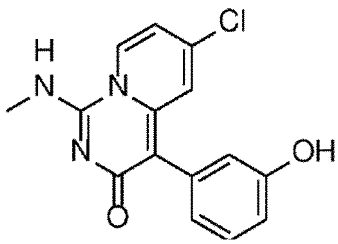 |
| **2.010** | 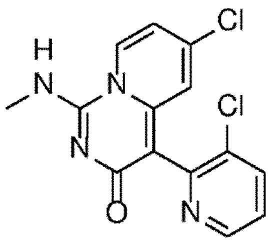 |
| **2.011** | 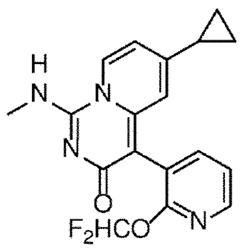 |
| **2.012** | 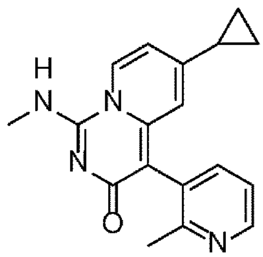 |

(continued)

| Composé numéro | Structure |
|---|---|
| **2.013** | 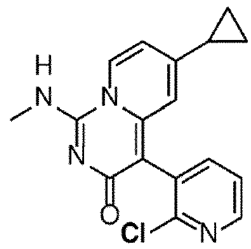 |
| **2.014** | 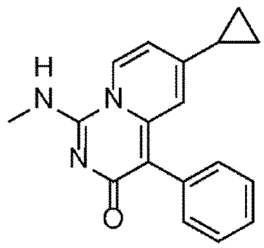 |
| **2.015** | 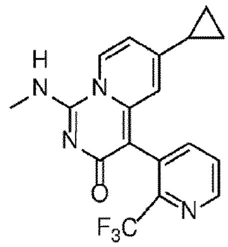 |
| **2.016** | 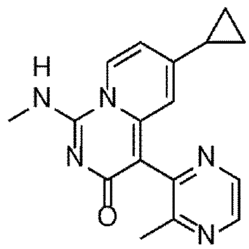 |
| **2.017** | 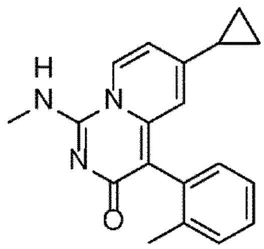 |
| **2.018** | 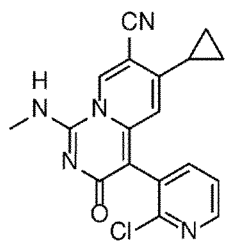 |

(continued)

| Composé numéro | **Structure** |
|---|---|
| **2.019** | 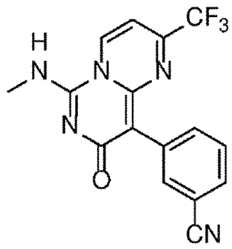 |
| **2.020** | 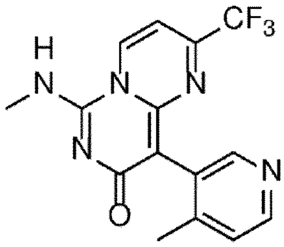 |
| **2.021** | 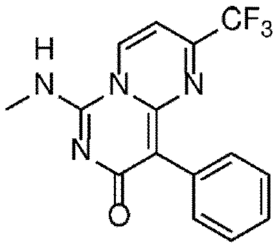 |
| **2.022** | 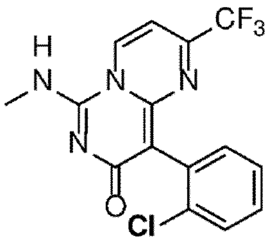 |
| **2.023** | 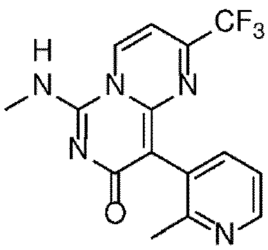 |
| **2.024** | 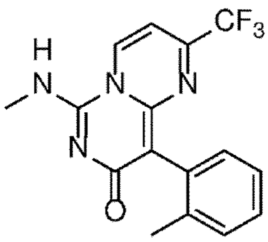 |

(continued)

| Composé numéro | **Structure** |
|---|---|
| **2.025** | 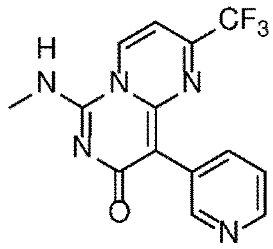 |
| **2.026** | 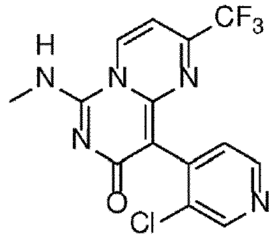 |
| **2.027** | 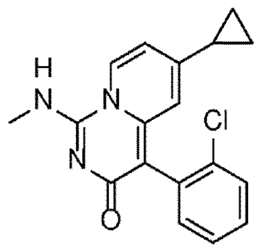 |

ou un sel pharmaceutiquement acceptable de ceux-ci.

**13.** Composition pharmaceutique comprenant un composé selon l'une quelconque des revendications 1 à 12, ou un sel pharmaceutiquement acceptable de celui-ci et au moins un excipient pharmaceutiquement acceptable.

**14.** Composé selon l'une quelconque des revendications 1 à 12, pour une utilisation dans un procédé de traitement d'une maladie médiée par MAT2A chez un patient comprenant l'administration au patient d'une quantité thérapeutiquement efficace de : le composé, ou le sel pharmaceutiquement acceptable de celui-ci, dans lequel la maladie est un cancer, de préférence dans lequel le cancer est choisi dans le groupe consistant en une leucémie, un gliome, un mélanome, un cancer du pancréas, un cancer du poumon non à petites cellules, un cancer de la vessie, un astrocytome, un ostéosarcome, un cancer de la tête et du cou, un chondrosarcome myxoïde, un cancer de l'ovaire, un cancer de l'endomètre, un cancer du sein, un sarcome des tissus mous, un lymphome non hodgkinien et un mésothéliome.

**15.** Composé selon l'une quelconque des revendications 1 à 10, ou un sel pharmaceutiquement acceptable de celui-ci, pour une utilisation dans un procédé de traitement d'un cancer MTAP-négatif chez un patient comprenant l'administration au patient d'une quantité thérapeutiquement efficace du composé ; ou du sel pharmaceutiquement acceptable de celui-ci, facultativement dans une composition pharmaceutique,
de préférence dans lequel le cancer est choisi dans le groupe consistant en une leucémie, un gliome, un mélanome, un cancer du pancréas, un cancer du poumon non à petites cellules, un cancer de la vessie, un astrocytome, un ostéosarcome, un cancer de la tête et du cou, un chondrosarcome myxoïde, un cancer de l'ovaire, un cancer de l'endomètre, un cancer du sein, un sarcome des tissus mous, un lymphome non hodgkinien et un mésothéliome.

**16.** Composé selon l'une quelconque des revendications 1 à 10, ou un sel pharmaceutiquement acceptable de celui-ci, pour une utilisation dans un procédé de traitement d'un cancer chez un patient, dans lequel le cancer est **caractérisé par** une réduction ou une absence d'expression du gène MTAP, l'absence du gène MTAP, un taux réduit de protéine MTAP, ou une fonction réduite de la protéine MTAP, comprenant l'administration au sujet d'une quantité thérapeutiquement efficace du composé, ou du sel pharmaceutiquement acceptable de celui-ci, facultativement dans une composition pharmaceutique,

de préférence dans lequel le cancer est choisi dans le groupe consistant en une leucémie, un gliome, un mélanome, un cancer du pancréas, un cancer du poumon non à petites cellules, un cancer de la vessie, un astrocytome, un ostéosarcome, un cancer de la tête et du cou, un chondrosarcome myxoïde, un cancer de l'ovaire, un cancer de l'endomètre, un cancer du sein, un sarcome des tissus mous, un lymphome non hodgkinien et un mésothéliome.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63037109
- WO 2018039972 A
- WO 2019191470 A

### Non-patent literature cited in the description


- **MCDONALD**. *Cell*, 2017, vol. 170, 577-592
- **MARJON**. *Cell Reports*, 2016, vol. 15 (3), 574-587
- **BERGE, S.M et al.** Pharmaceutical Salts. *Journal of Pharmaceutical Science*, 1977, vol. 66, 1-19
- **T.W. GREENE**. Protective Groups in Organic Synthesis. John Wiley & Sons, Inc, 2014
- Fieser and Fieser's Reagents for Organic Synthesis. John Wiley and Sons, 1991, vol. 1-17
- *Rodd's Chemistry of Carbon Compounds*, vol. 1-5
- Supplementals. Elsevier Science Publishers, 1989
- Organic Reactions. John Wiley and Sons, 1991, vol. 1-40
- March's Advanced Organic Chemistry. John Wiley and Sons
- Larock's Comprehensive Organic Transformations. VCH Publishers Inc, 1989
- **K. MARJON et al.** *Cell Reports*, 2016, vol. 15, 574-587